# EUROPEAN PATENT SPECIFICATION

(11) **EP 0 508 687 B1**
(45) Date of publication and mention of the grant of the patent: **13.09.1995**
(21) Application number: 92302927.6
(22) Date of filing: 02.04.1992
(51) Int. Cl.: C07H 19/20, A61K 31/70, C07D 473/00

(54) **ATP analogues**
ATP-Analogen
Analogues d'ADP

(30) Priority: 06.04.1991 GB 9107236; 07.11.1991 GB 9123671
(43) Date of publication of application: 14.10.1992
(73) Proprietor: Astra Pharmaceuticals Limited, Kings Langley, Herts WD4 8DH (GB)
(72) Inventor: Ingall, Anthony Howard, Leicestershire LE11 3NW (GB); Cage, Peter Alan, Leicestershire LE12 9BZ (GB)
(74) Representative: Wright, Robert Gordon McRae

(56) References cited:
- WO-A-90/11080
- CHEMICAL ABSTRACTS, vol. 107, no. 1, 6 July 1987, Columbus, Ohio, US; abstract no. 258T, N.J.CUSACK ET AL.: 'Pharmacological Effects of Isopolar Phosphonate Analogs of ATP on P2-purinoceptors in Guinea Pig Tenia Coli and Urinary Bladder' page 251 ;column 2 ;
- CHEMICAL ABSTRACTS, vol. 108, no. 15, 11 April 1988, Columbus, Ohio, US; abstract no. 125109X, C.J.TSENG ET AL.: 'Purinergic Receptors in the Brainstem Mediate Hypotension and Bradycardia' page 124 ;column 1 ;
- CHEMICAL ABSTRACTS, vol. 92, no. 15, 14 April 1980, Columbus, Ohio, US; abstract no. 122602T, M.H.MAGUIRE ET AL.: 'Specificity of Adenine Nucleotide Receptor Sites: Inhibition of the Guinea Pig Taenia Coli by Adenine Nucleotide Analogs' page 135 ;column 2 ;
- BIOCHIMIE vol. 72, no. 10, October 1990, pages 719 - 724; A.G.RABINKOV ET AL.: 'Interaction of ATP with Acetyl CoA Carboxylase from Rat Liver. The Role of the Polyphosphate Chain, Affinity Labelling with Alkylating Amides of ATP and ADP'
- JOURNAL OF MEDICINAL CHEMISTRY. vol. 16, no. 10, October 1973, WASHINGTON US pages 1188 - 1190; G.R.GOUGH ET AL.: 'Three New Triphosphate Analogs. Synthesis and Effects on Isolated Gut'
- NUCLEOSIDES AND NUCLEOTIDES vol. 10, no. 5, 1991, pages 1019 - 1028; N.J.CUSACK ET AL.: 'Design, Syntheses and Pharmacology of ATP Analogues Selective for Subtypes of P2-Purinoceptors'
- NUCLEOSIDES AND NUCLEOTIDES vol. 10, no. 1-3, 1991, pages 549 - 551; G.M.BLACKBURN ET AL.: 'Symthesis, Physical, Chemical and Enzyme Studies on Bis-2,6-Diaminopurine B-D-Ribofuranoside P1,P4-Tetraphosphate'
- G. Gough et al. Molecular Pharmacology 1972, Vol.8(, p. 170-177
- Cusack et al. Mechanism of Stimulus- response Coupling in Platelets 1985, p. 29-39

## Description

This invention relates to pharmaceutically useful compounds, and processes for their production.

Adenosine triphosphate (ATP) has potent pharmacological effects on a variety of tissues, the activity of ATP and the other extracellular adenosine nucleotides, adenosine diphosphate (ADP) and adenosine monophosphate (AMP), are mediated by P₂-purinoceptors. However, the potency of ATP in some tissues, e.g. the bladder, may be reduced due to rapid dephosphorylation, to AMP and adenosine, by ectonucleotidases present in these tissues.

In recent studies ATP analogues which are resistant to dephosphorylation have been used as biological probes to investigate the P₂-purinoceptors present in a variety of tissues:
Cusack *et al*, *Br. J. Pharmacol.*, 1987, **90**, 791-795, describe the activity of 2-methylthio-5'-adenylic acid, monoanhydride with methylenebisphsophonic acid, 2-methylthio-5'-adenylic acid, monoanhydride with dichloromethylenebisphosphonic acid and 2-methylthio-5'-adenylic acid, monoanhydride with difluoromethylenebisphosphonic acid on the guinea pig taenia coli and urinary bladder. Stone and Cusack, *Br. J. Pharmacol*, 1989, **97**, 631-635, describe the use of *inter alia* 2-methylthio-5'-adenylic acid, monoanhydride with difluoromethylenebisphosphonic acid in an investigation of P₂-purinoceptors in the rat hippocampus. Gough *et al*, *J. Med. Chem.*, 1973, **16**(10), 1188-1190; and Maguire and Satchell in "Physiological and Regulatory Functions of Adenosine and Adenine Nucleotides", Ed. H.P. Baer and G.I. Drummond, Raven Press, New York, 1979, p.33-43, disclose the inhibition of guinea pig taenia coli by the compound 2-chloro-5'-adenylic acid, monoanhydride with methylenebisphophonic acid.

Blackburn and Guo, *Nucleosides & Nucleotides*, 1991, **10**(1-3), 549-551, disclose monoanhydrides of adenylic acid with several methylenebisphosphonic acids, however no pharmaceutical utility is suggested for these compounds.

Gough *et al*, *Molecular Pharmacology*, 1972, **8**, 170-177, disclose an investigation into the platelet aggregating potency of various analogues of ADP. The compound 2-chloro-adenosine 5'-methylene diphosphonate is stated to be devoid of aggregating activity. Furthermore, since it does not act as a competitive inhibitor of ADP it is concluded that it does not bind to the receptor.

Cusack *et al*, in "Mechanisms of Stimulus-Response Coupling in Platelets", Ed. J. Westwick, D.E. MacIntyre, M.F. Scully and V.V. Kakkar, Plenum, New York, 1985, p.29-39, report that 2-methylthio-5'-adenylic acid, monoanhydride with methylenebisphosphonic acid is a specific but non-competitive inhibitor of ADP induced platelet aggregation. Cusack and Hourani, *Nucleosides & Nucleotides*, 1991, **10**(5), 1019-1028, have also reported that 2-methylthio-5'-adenylic acid, monoanhydride with methylenebisphosphonic acid inhibits ADP-β-S induced platelet aggregation.

We have now found a group of novel 2-substituted ATP derivatives which exhibit pharmacological activity.

According to a first aspect of the present invention, there is provided a compound of formula I, 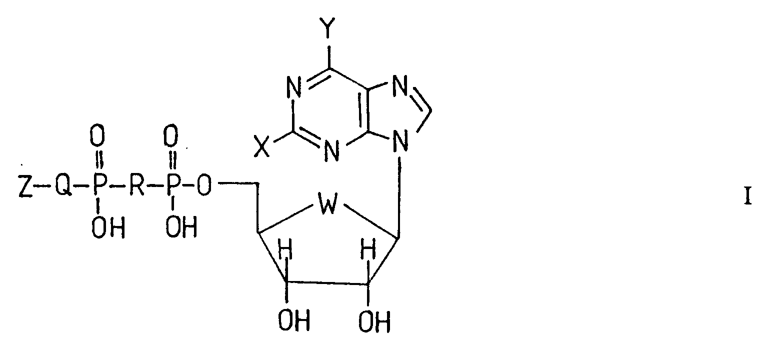
wherein Q represents CR¹R²,
R represents O or CR³R⁴,
W represents O or CH₂,
R¹, R², R³ and R⁴ independently represent hydrogen or halogen,
X represents S(O)ₙR⁵,
n represents 0, 1 or 2,
R⁵ represents ethyl, butyl or propyl,
Y represents NH₂ or alkoxy C₁₋₆, and
Z represents an acidic moiety,
in addition:
a) when R represents CR³R⁴, then -Q-Z may represent hydroxy or -OP(O)(OH)₂; and
b) when R and W represent O, Q represents CCl₂ or CBr₂, Y represents NH₂ and Z represents -P(O)(OH)₂, then X may represent S-pentyl; and pharmaceutically acceptable salts thereof.

Compounds of formula I may exist in tautomeric, enantiomeric and diastereomeric forms, all of which are included within the scope of the invention.

According to the invention there is further provided a process for the preparation of compounds of formula I, and salts thereof, which comprises:
a) producing a compound of formula I in which R represents O, or a salt thereof, by reacting a compound of formula II, or a salt thereof, 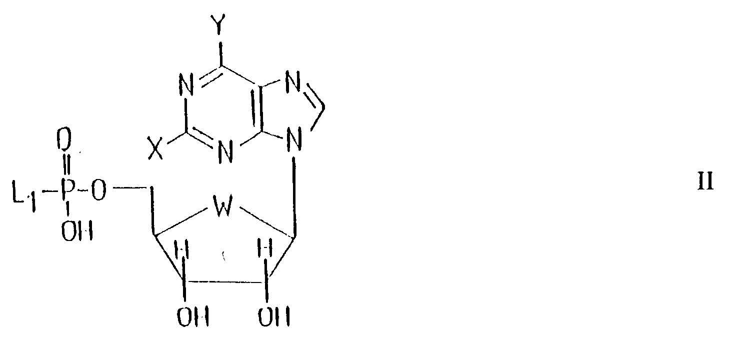 wherein W, X and Y are as defined above and L₁ represents a leaving group, with a compound of formula III, or a salt thereof, 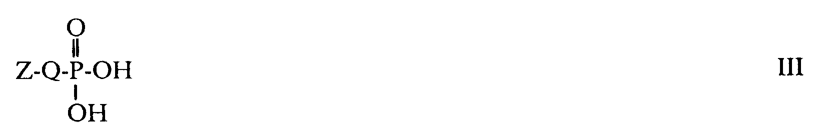 wherein Z and Q are as defined above.
b) producing a compound of formula I in which R represents CR³R⁴, or a salt thereof, by reacting a compound of formula IV, or a salt thereof, 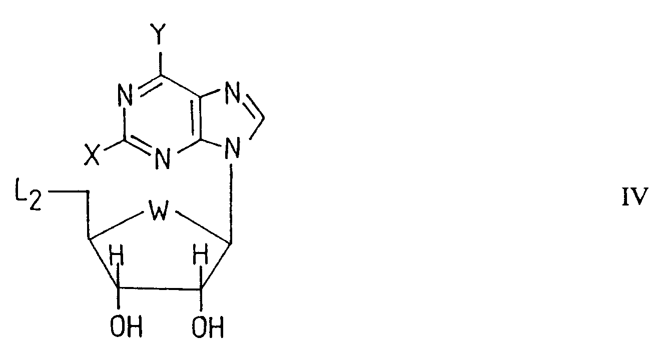 wherein W, X and Y are as defined above and L₂ represents a leaving group, with a compound of formula V, or a salt thereof, 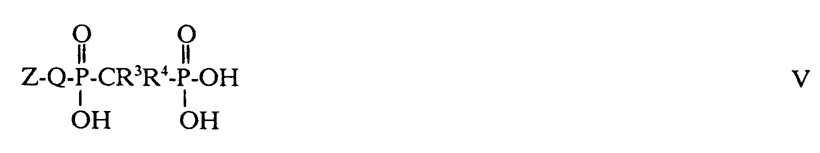 wherein Z, Q, R³ and R⁴ are as defined above.
c) producing a compound of formula I in which R is CR³R⁴ and -Q-Z is -OP(O)(OH)₂, or a salt thereof, by reacting a corresponding compound of formula I in which -Q-Z is hydroxy with a compound of formula VI, or a salt thereof, 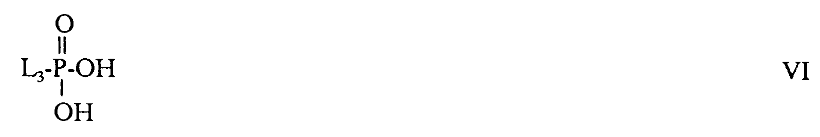 wherein L₃ is a leaving group.
d) removal of a protecting group from a corresponding protected compound of formula I in which one or more of the functional groups is protected,
   and where desired or necessary converting the resultant compound of formula I, or another salt thereof, to a pharmaceutically acceptable salt thereof or vice versa.

In processes a) and c) leaving groups which L₁, and L₃ may represent include amines, for example, dialkylamines or saturated or unsaturated cyclicamines; particular leaving groups which may be mentioned include morpholinyl, imidazolyl and triazolyl.

In process b) leaving groups which L₂ may represent include alkyl or arylsulphonyloxy groups such as methane-sulphonyloxy, trifluoromethanesulphonoxy or p-toluene-sulphonyloxy; or trifluoroacetoxy.

In processes a), b) and c) the solvent used is preferably a dipolar aprotic solvent, for example, pyridine, dimethylformamide, acetonitrile, hexamethylphosphorictriamide, N,N'-dimethylpropyleneurea or 1-methyl-2-pyrrolidinone. The reaction may be carried out at a temperature of from -20 to 100°C, e.g. from 10 to 30°C.

Compounds of formulae II to VI are either known or may be prepared using methods known to those skilled in the art or by techniques analogous to those given in the examples. For example, compounds of formula II in which L₁ represents morpholinyl may be prepared from the corresponding 5'-monophosphates by treatment with morpholine in the presence of a condensing agent such as dicyclohexylcarbodiimide, preferably in the presence of a protic solvent or mixture of solvents such as ^{t}butanol and water.

For compounds in which W is O, the nucleoside 5'-monophosphates and nucleosides used in the preparation of the compounds of formulae II and IV respectively are either known or may be prepared from known compounds using known techniques, see, for example, "Chemistry of Nucleosides and Nucleotides" Vol. 2, Ed. Leroy B. Townsend, Plenum Press 1991.

In the above processes it may be necessary for any functional groups, e.g. hydroxy or amino groups, present in the starting materials to be protected, thus process d) may involve the removal of one or more protecting groups. Suitable protecting groups and methods for their removal are, for example, those described in "Protective Groups in Organic Chemistry" by Theodora Greene, John Wiley and Sons Inc., 1981. Hydroxy groups may, for example, be protected by arylmethyl groups such as phenylmethyl, diphenylmethyl or triphenylmethyl, or as tetrahydropyranyl derivatives. Suitable amino protecting groups include arylmethyl groups such as benzyl, (R,S)-α-phenylethyl, diphenylmethyl or triphenylmethyl, and acyl groups such as acetyl, trichloroacetyl or trifluoroacetyl. Conventional methods of deprotection may be used including hydrogenolysis, acid or base hydrolysis, or photolysis. Arylmethyl groups may, for example, be removed by hydrogenolysis in the presence of a metal catalyst e.g. palladium on charcoal. Tetrahydropyranyl groups may be cleaved by hydrolysis under acidic conditions. Acyl groups may be removed by hydrolysis with a base such as sodium hydroxide or potassium carbonate, or a group such as trichloroacetyl may be removed by reduction with, for example, zinc and acetic acid.

Salts of the compounds of formula I may be formed by reacting the free acid, or a salt thereof, or the free base, or a salt or derivative thereof, with one or more equivalents of the appropriate base or acid. The reaction may be carried out in a solvent or medium in which the salt is insoluble or in a solvent in which the salt is soluble, e.g. ethanol, tetrahydrofuran or diethyl ether, which may be removed *in vacuo*, or by freeze drying. The reaction may also be a metathetical process or it may be carried out on an ion exchange resin.

Pharmaceutically acceptable salts of the compounds of formula I include alkali metal salts, e.g. sodium and potassium salts; alkaline earth metal salts, e.g. calcium and magnesium salts; salts of the Group III elements, e.g. aluminium salts; and ammonium salts. Salts with suitable organic bases, for example, salts with hydroxylamine; lower alkylamines, e.g. methylamine or ethylamine; with substituted lower alkylamines, e.g. hydroxysubstituted alkylamines; or with monocyclic nitrogen heterocyclic compounds, e.g. piperidine or morpholine; and salts with amino acids, e.g. with arginine, lysine etc, or an N-alkyl derivative thereof; or with an aminosugar, e.g. N-methyl-D-glucamine or glucosamine. The non-toxic physiologically acceptable salts are preferred, although other salts are also useful, e.g. in isolating or purifying the product.

Alkyl groups include straight, branched or cyclic, saturated or unsaturated alkyl groups.

Aryl groups include both carbocyclic and heterocyclic groups. The groups may contain rings of various numbers of C-atoms and may be fused ring structures. Particular carbocyclic aryl groups which may be mentioned are phenyl and naphthyl. Heteroaryl groups include nitrogen, oxygen or sulphur heterocycles and may contain one or more heteroatoms. Examples of heterocycles containing only one heteroatom include pyrrole, furan, thiophen and pyridine. Groups containing more than one heteroatom include pyrazole, oxazole, thiazole, triazole, oxadiazole, thiadiazole etc.

Halogens which X, R¹, R², R³ and R⁴ may represent include F, Cl, Br and I.

When Q represents CR¹R², we prefer R¹ and R² to be the same, we particularly prefer compounds in which R¹ and R² both represent Cl.

When R represents CR³R⁴, we prefer R³ and R⁴ to be the same, we particularly prefer compounds in which R³ and R⁴ both represent hydrogen or Cl.

We prefer compounds in which Q represents CR¹R².

We prefer compounds in which R represents O.

We particularly prefer compounds of formula I in which Q represents CR¹R² and R represents O.

We prefer compounds in which W represents O.

We prefer compounds of formula I in which n represents 0. We prefer compounds of formula I in which R⁵ represents n-propyl.

We prefer compounds in which Y represents NH₂.

Acidic moieties which Z may represent include Bronsted-Lowry acids, i.e. moieties which act as proton donors. The acidic moiety may be mono- or poly-acidic. Specific acidic moieties which may be mentioned include -P(O)(OH)₂, -SO₃H and -CO₂H.

We prefer compounds of formula I in which Z represents -P(O)(OH)₂.

The compounds of formula I are useful because they exhibit pharmacological activity in mammals. In particular, they show activity in the prevention of platelet aggregation.

The potency of the compounds of formula I as inhibitors of platelet aggregation may be determined from their ability to act as P_{2T} receptor antagonists, see Example X.

The compounds may be used in any condition where platelet aggregation or disaggregation is involved. The compounds may thus act as anti-thrombotic agents and are indicated in the treatment or prophylaxis of unstable angina, thromboembolic stroke and peripheral vascular disease. They are also indicated in the treatment or prophylaxis of the sequelae of thrombotic complications from angioplasty, thrombolysis, endarterctomy, coronary and vascular graft surgery, renal dialysis and cardio-pulmonary bypass. Further indications include the treatment or prophylaxis of disseminated intravascular coagulation, deep vein thrombosis, pre-eclampsia/eclampsia, tissue salvage following surgical or accidental trauma, vasculitis, arteritis, thrombocythaemia, ischemia and migraine.

According to a further aspect of the invention, we therefore provide the compounds of formula I, as defined above, as pharmaceuticals.

The dosage to be administered will vary widely, depending on, amongst other factors, the particular compound of formula I employed, the particular condition to be treated and its severity. However, in general a total daily dose of 1g may be suitable, which may be administered in divided doses up to 6 times per day.

The compounds will generally be administered in the form of a pharmaceutical composition.

Thus, according to a further aspect of the invention there is provided a pharmaceutical composition comprising preferably less than 80% w/w, more preferably less than 50% w/w, e.g. 0.1 to 20%, of a compound of formula I, or a pharmaceutically acceptable salt thereof, as defined above, in admixture with a pharmaceutically acceptable adjuvant, diluent or carrier.

Examples of pharmaceutical formulations which may be used, and suitable adjuvants, diluents or carriers, are as follows:
for intravenous injection or infusion - purified water or saline solution;
for inhalation compositions - coarse lactose;
for tablets, capsules and dragees - microcrystalline cellulose, calcium phosphate, diatomaceous earth, a sugar such as lactose, dextrose or mannitol, talc, stearic acid, starch, sodium bicarbonate and/or gelatin;
for suppositories - natural or hardened oils or waxes.

When the compound is to be used in aqueous solution, e.g. for infusion, it may be necessary to incorporate other excipients. In particular there may be mentioned chelating or sequestering agents, antioxidants, tonicity adjusting agents, pH-modifying agents and buffering agents.

Solutions containing a compound of formula I may, if desired, be evaporated, e.g. by freeze drying or spray drying, to give a solid composition, which may be reconstituted prior to use.

When not in solution, the compound of formula I preferably is in a form having a mass median diameter of from 0.01 to 10µm. The compositions may also contain suitable preserving, stabilising and wetting agents, solubilisers, e.g. a water-soluble cellulose polymer such as hydroxypropyl methylcellulose, or a water-soluble glycol such as propylene glycol, sweetening and colouring agents and flavourings. Where appropriate, the compositions may be formulated in sustained release form.

According to a further aspect of the invention we therefore provide the use of a compound of formula I, or a pharmaceutically acceptable salt thereof, in the manufacture of a pharmaceutical composition for the treatment of a condition where platelet aggregation or disaggregation is involved.

According to a further aspect of the invention, we therefore provide a method of treating a condition where platelet aggregation or disaggregation is involved which comprises administering a therapeutically effective amount of a compound of formula I, as defined above to a patient suffering from such a condition.

The compounds of the invention are advantageous in that they are less toxic, more efficacious, are longer acting, have a broader range of activity, are more potent, are more stable, produce fewer side effects, are more easily absorbed, are more readily cleared from the body or have other useful pharmacological properties, than compounds previously used in the therapeutic fields mentioned above.

The invention is illustrated by the following Examples, in which temperatures are given in degrees Celsius. Examples are named using Chemical Abstracts nomenclature.

### Example 1

### 2-Propylthio-5'-adenylic acid monoanhydride with dibromomethylenebisphosphonic acid, tetrasodium salt

i) 2-Propylthioadenosine
   Adenosine-2-thione (2.5 g) in water (15 ml), methanol (45 ml) and 1N sodium hydroxide solution (8.36 ml) were cooled in an icebath and iodopropane (5 ml) introduced. After 4 days the reaction mixture was evaporated and the residue chromatographed (SiO₂, ethyl acetate:methanol, 9:1) to afford the sub-title compound in quantitative yield.
   NMR δ¹H (d⁶DMSO) 8.22 (s, 1H), 7.36 (brs, 2H), 5.80 (d, 1H, J=5.9Hz), 5.61 (t, 1H, J=5.5Hz), 5.13 (m, 1H), 3.90 (dd, 1H, J=4.0 and 7.7Hz), 3.64 (dd, 1H, J=4.2 and 11.8Hz), 3.52 (dd, 1H, J=4.4 and 11.8Hz), 3.0-3.2 (m, 2H), 1.67 (hextet, 2H, J=7.3Hz), 0.99 (t, 3H, J=7.3Hz).
ii) 2-Propylthio-5'-adenylic acid
   The product of step i) (1.0 g) was added to a stirred mixture of phosphorus oxychloride (1.06 ml) and triethyl phosphate (25 ml) at -10°C. After 3 hours the reaction mixture was poured onto ice and the pH adjusted to 7 with solid sodium bicarbonate. The solution was washed with ether (3x150 ml) and then lyophilised. The resulting solid was taken in deionised water and applied to a column of Dowex 50Wx8 (H⁺ form), which was washed with water until the eluate was at pH 6, then eluted with 1M ammonium hydroxide. Lyophilisation afforded the sub-title compound (0.4 g).
   NMR δ³¹P (D₂O) 1.32 (s).
iii) 2-Propylthio-5'-adenylic acid, monoanhydride with dibromomethylenebisphosphonic acid, tetrasodium salt
   The product of step ii) (0.4 g) and tri-n-butylamine (0.175 g) were combined in a small volume of water and the solution evaporated to dryness. Azeotropic drying with pyridine (3x15 ml) followed by anhydrous dimethylformamide (2x15 ml) left a residue which was taken into anhydrous dimethylformamide (10 ml). Carbonyldiimidazole (0.77 g) was added, and the reaction left at room temperature for 4 hours before adding methanol (0.24 g). After 30 min the mono tri-n-butylammonium salt of dibromomethylenebisphosphonic acid (6.5 mmol) in anhydrous dimethylformamide (30 ml) was added, and the mixture stirred at room temperature for 18 hours. Filtration and evaporation afforded a residue which was purified by chromatography (DEAE-Sepharose, aqueous ammonium bicarbonate 0-0.4M as eluant). Lyophilisation gave the ammonium salt which was redissolved in water (200 ml) and treated with triethylamine (10 ml). Evaporation *in vacuo* produced the tetrakistriethylammonium salt which was converted to the tetrasodium form by dissolution in methanol (2 ml) and addition of sodium iodide solution (1M in acetone, 30 ml). The precipitate was collected by centrifugation, washing by repeated suspension in acetone (4x40 ml) and recentrifugation. Finally the solid was dissolved in water and lyophilised to afford the title salt as a white powder (0.42 g).
   NMR δ³¹P (D₂O) 8.95 (d, J=36Hz), 1.35 (dd, J=36 and 69Hz), -9.15 (d, J=69Hz).

### Example 2

The following compounds were prepared according to the method of

### Example 1:

a) 2-Propylthio-5'-adenylic acid, monoanhydride with difluoromethylenebisphosphonic acid, tetrasodium salt
   NMR δ³¹P (D₂O) 3.94 (dt, J=145.8 and 196Hz), 3.11 to -4.92 (m), -10.15 (d, J=75Hz).
b) 2-Propylthio-5'-adenylic acid, monoanhydride with dichloromethylenebisphosphonic acid, tetrasodium salt
   NMR δ³¹P (D₂O) 9.4 (d, J=46Hz), 2.3 (dd, J=46 and 73.1Hz), -9.15 (d, J=73.1Hz).
c) 2-Pentylthio-5'-adenylic acid, monoanhydride with dichloromethylenebisphosphonic acid, tetrasodium salt
   i) 2-Pentylthioadenosine
      NMR δ¹H (d⁶DMSO) 8.22 (s, 1H), 7.35 (brs, 1H), 5.80 (d, 1H, J=5.9Hz), 5.42 (d, 1H, J=6.16Hz), 5.16 (d, 1H, J=4.86Hz), 5.02 (t, 1H, J=5.6Hz), 4.61 (q, 1H, J=5.76Hz), 4.07-4.15 (m, 1H), 3.85-3.95 (m, 1H), 3.6-3.7 (m, 1H), 3.48-3.6 (m, 1H), 3.0-3.15 (m, 2H), 1.6-1.7 (m, 2H), 1.3-1.45 (m, 4H), 0.88 (t, 3H, J=7.02Hz).
   ii) 2-Pentylthio-5'-adenylic acid
      NMR δ³¹P (D₂O) 1.2 (s).
   iii) 2-Pentylthio-5'-adenylic acid, monoanhydride with dichloromethylenebisphosphonic acid, tetrasodium salt
      NMR δ³¹P (D₂O) 8.55 (d, J=46.2Hz), 2.5-3.5 (m), -9.12 (d, J=70.34Hz).
d) 2-Pentylthio-5'-adenylic acid, monoanhydride with dibromomethylenebisphosphonic acid, monoammonium salt
   Purification of the crude product by chromatography (DEAE-Sepharose, ammonium bicarbonate 0-0.4M as eluant) afforded the title salt.
   NMR δ³¹P (D₂O) 7-8 (brs), 2.5-3.5 (m), -9.07 (d, J=83.5Hz).
e) 2-Ethylthio-5'-adenylic acid, monoanhydride with dichloromethylenebisphosphonic acid, tetrasodium salt
   i) 2-Ethylthio-5'-adenylic acid, disodium salt
      NMR δ³¹P (D₂O) 5.01 (s).
   ii) 2-Ethylthio-5'-adenylic acid, monoanhydride with dichloromethylenebisphosphonic acid, tetrasodium salt
      NMR δ³¹P (D₂O) 8.25 (d, J=17.04Hz), 3.28 (dd, J=18.9 and 28.22Hz), -9.22 (d, J=28.22Hz).
f) 2-Ethylthio-5'-adenylic acid, monoanhydride with dibromomethylenebisphosphonic acid, tetrasodium salt
   NMR δ³¹P (D₂O) 6.5-7.5 (m), 2.88 (dd, J=14.75 and 27.33Hz), -10.9 (d, J=28.22Hz).
g) 2-Butylthio-5'-adenylic acid, monoanhydride with dichloromethylenebisphosphonic acid, monoammonium salt
   i) 2-Butylthio-5'-adenylic acid
      NMR δ³¹P (D₂O) 1.84 (s).
   ii) 2-Butylthio-5'-adenylic acid, monoanhydride with dichloromethylenebisphosphonic acid, monoammonium salt
      NMR δ³¹P (D₂O) 8.05-8.25 (m), 3.66 (dd, J=18.48 and 27.8Hz), -9.03 (d, J=27.8Hz).
h) 2-Propylthio-5'-adenylic acid, monoanhydride with methylenebisphosphonic acid, tetrasodium salt
   NMR δ³¹P (D₂O) 15.8 (d, J=8.3Hz), 10.7 (dd, J=8.4 and 27Hz), -9.44 (d, J=25.8Hz).
i) 2-Propylthio-5'-adenylic acid, monoanhydride with sulphodifluoromethylphosphonic acid, trisodium salt
   NMR δ³¹P (D₂O) -8.8 (dt), -10.0 (d).
j) 2-Propylthio-5'-adenylic acid, monoanhydride with phosphonoacetic acid, trisodium salt
   NMR δ³¹P (D₂O) 11.4 (d), -9.57 (d).

### Example 3

### 2-(1-Methylethyl)thio-5'-adenylic acid, monoanhydride with dichloromethylenebisphosphonic acid, trisodium salt

i) 6-Chloro-2-[(1-methylethyl)-thio]-9-(2,3,5-tri-O-acetyl-β-D-ribofuranosyl)-9H-purine
   9-(2',3',5'-Tri-O-acetyl-β-D-ribofuranosyl)-2-amino-6-chloropurine (6.6 g) was dissolved in dry acetonitrile (65 ml). Isopropyl disulphide (21 ml) and isoamyl nitrite (12.1 ml) were added and the resulting solution purged with nitrogen for 45 min, then heated under nitrogen at 60° for 16 hours. The volatiles were removed *in vacuo* and the residue chromatographed to afford the sub-title compound as a yellow oil (3.69 g).
   NMR δ¹H (d₆-DMSO) 8.70 (s, 1H), 6.29 (d, 1H), 6.02 (m, 1H), 5.61 (t, 1H), 4.39 (m, 2H), 4.20 (m, 1H), 4.00 (septet, 1H), 2.12, 2.07, 1.98 (3xs, 3x1H), 1.41 d, 6H).
ii) 2-(1-Methylethyl)thio-adenosine
   The product of step i) (3.6 g) in ethanol (400 ml) was cooled to 0° and saturated with ammonia. The solution was allowed to warm to approx. 15°, then heated to 70° in an autoclave for 24 hours, the volatiles were removed *in vacuo* and the residue chromatographed to afford the sub-title compound (1.95 g).
   MS (FAB) 342 (M⁺+H) (100%).
iii) 2-(1-Methylethyl)thio-5'-adenylic acid
   The sub-title compound was prepared according to the method of Example 1.
   NMR δ³¹P (D₂O) 1.1 (s).
iv) 2-(1-Methylethyl)thio-5'-adenylic acid, monoanhydride with dichloromethylenebisphosphonic acid, trisodium salt
   The title compound was prepared according to the method of Example 1.
   NMR δ³¹P (D₂O) 9.21 (d, J=21Hz), 2.0 (dd, J=19 and 30Hz), -9.33 (d, J=32Hz).

### Example 4

### 2-Propylthioadenosine, 5'-P¹ monoester with bis[(dihydroxyphosphinyl)methyl]phosphinic acid, disodium salt

i) 2',3'-O,O-(1-Methylethylidene)-2-propylthioadenosine
   To a suspension of 2-propylthioadenosine (2.6 g) in AR acetone (97 ml) and 2,2-dimethoxypropane (11.3 ml) was added p-toluenesulphonic acid monohydrate (1.46 g) in small portions over 1 hour. The resulting solution was stirred at ambient temperature for 18 hours, diluted with water (300 ml) and treated with triethylamine to pH 7. The volume was reduced to half *in vacuo*, and the remaining solution extracted into chloroform (3x100 ml). The extract was dried (MgSO₄), filtered and evaporated. The residue was purified by chromatography (SiO₂, ethyl acetate) to afford the sub-title compound (1.8 g).
   MS (FAB) 382 (M⁺+H), BP 210.
ii) 5'-4-Methylbenzenesulphonyl-2',3'-O,O-(1-methylethylidene)-5'-deoxy-2-propylthioadenosine
   To an ice-cooled solution of the product of step i) (1.6 g) in dry dichloromethane (100 ml) was added 4-dimethylaminopyridine (1.33 g) and then a solution of p-toluenesulphonyl chloride (0.88 g) in dry dichloromethane (20 ml) over 15 min. After 18 hours at 0-4° the volatiles were removed *in vacuo* and the residue purified by chromatography (SiO₂, ethyl acetate) to afford the sub-title compound (1.9 g).
   NMR δ¹H (CDCl₃) 7.7 (s, 1H), 7.6 (d, 2H), 7.1 (d, 2H), 6.0 (d, 1H), 5.7 (s, 2H), 5.4 (d, 1H), 5.0 (m, 1H), 4.4 (m, 1H), 4.2 (m, 2H), 3.0 (m, 2H), 2.4 (s, 3H), 1.7 (q, 2H), 1.6 (s, 3H), 1.4 (s, 3H), 1.1 (t, 3H).
iii) 2-Propylthioadenosine, 5'-P¹ monoester with bis[(dihydroxyphosphinyl)methyl]phosphinic acid, disodium salt
   A solution of bis[(dihydroxyphosphinyl)methyl]phosphinic acid (0.66 g) in water (10 ml) was adjusted to pH 8.7 by addition of 40% w/v tetrabutylammonium hydroxide solution, then lyophilised to afford a gum, which was further dried by repeated dissolution in acetonitrile and evaporation of the solvent (3x50 ml). The residue was taken in dry acetonitrile (10 ml) and treated with the product of step ii) (0.81 g) and the mixture concentrated *in vacuo* to a thick syrup. After stirring overnight at ambient temperature the mixture was diluted with water and applied to a column of DEAE-Sepharose (Fast Flow). Elution with 0-0.6M triethylammonium bicarbonate afforded, after lyophilisation, a residue which was dissolved in 80% v/v acetic acid and heated to 80° for 4 hours. After evaporation to dryness the residue was azeotroped with water (2x20 ml), dissolved in methanol (5 ml) and treated with a few drops of triethylamine. To this solution was added 1M sodium iodide in acetone to precipitate the sodium salt, which was collected by centrifugation, and washed several times with acetone. The solid was taken up in water and freeze-dried to yield the title salt as a colourless solid (96 mg).

NMR δ³¹P (D₂O) 28.17 (d), 18.86 (dd), 16.39 (d).

### Example 5

### 2-Propylthioadenosine, 5'-P¹ monoester with bis[(dichloro(dihydroxyphosphinyl))methyl]phosphinic acid, tetrasodium salt

i) Bis[(dichloro(dihydroxyphosphinyl))methyl]phosphinic acid, pentaethyl ester
   A solution of bis[(dihydroxyphosphinyl)methyl]phosphinic acid pentaethyl ester (3.94 g) in chloroform (100 ml) was vigorously stirred with commercial sodium hypochlorite solution (5.25% available chlorine, 400 ml) at ambient temperature for 24 hours. The organic phase was separated and evaporated to afford the sub-title compound (4.7 g).
   MS (FAB) 531/533/535/537 (M⁺+H), BP 533.
ii) Bis[(dichloro(dihydroxyphosphinyl))methyl]phosphinic acid
   The product of step i) (0.532 g) in dry methylene chloride (10 ml) was treated with trimethylsilyl bromide (0.912 g) at ambient temperature for 18 hours. The volatiles were removed *in vacuo*, and the residue taken up in methanol (5 ml) which was evaporated after 2 hours, the resulting oil was taken up in deionised water (10 ml) and extracted well with ether. Cyclohexylamine was added until the pH was approx. 10, then methanol was added until a turbidity persisted. The solid which deposited on cooling was collected and dried, then taken up in water (10 ml) and applied to a Dowex 50Wx8 (H⁺) column. Elution with distilled water and lyophilisation afforded the sub-title compound (0.15 g).
   NMR δ³¹P (D₂O) 9.2 (d, J=20.4Hz), 17.9 (t, J=18.7Hz).
iii) 2-Propylthioadenosine, 5'-P¹ monoester with bis[(dichloro(dihydroxyphosphinyl))methyl]phosphinic acid, tetrasodium salt
   The title compound was prepared from the product of step ii) according to the method of Example 4 (0.35 g).
   NMR δ³¹P (D₂O) 20.81 (t, J=14.1Hz), 11.18 (d, J=12.1Hz), 9.9 (d, J=12.3Hz).

### Example 6

### 2-Propylthioadenosine, 5'-P¹ monoester with methylenebisphosphonic acid, P²-monoanhydride with phosphoric acid, tetrasodium salt

i) 2-Propylthioadenosine. 5'-P¹ monoester with methylenebisphosphonic acid, disodium salt
   The sub-title compound was prepared by the method of Example 4iii).
   NMR δ³¹P (D₂O) 20.97 (d, J=8.56Hz), 15.3 (d, J=8.57Hz).
ii) 2-Propylthioadenosine, 5'-P¹ monoester with methylenebisphosphonic acid, P²-monoanhydride with 1-(2-nitrophenyl)ethyl phosphate, monotriethylammonium salt
   The product of step i) was eluted through a column of Dowex 50Wx8 (n-Bu₄N⁺ form) with deionised water and the effluent was lyophilised to afford the tetra-n-butylammonium salt of the bisphosphonate material. This salt (0.73 mmol) and 1-(2-nitophenyl)ethyl phosphoromorpholidate, N,N'-dicyclohexyl morpholine-1-carboxamidinium salt (1.09 mmol) were stirred in dry pyridine (5 ml) for 48 hours at ambient temperature. The solvent was evaporated and the residue taken up in water (100 ml) and washed with chloroform (2x50 ml). The volume of the aqueous phase was reduced to 10 ml at 30° and applied to a column of DEAE-Sepharose which was eluted with 0-0.4M triethylammonium bicarbonate solution. The appropriate fractions were combined and lyophilised to afford the sub-title compound (0.3 g).
   NMR δ³¹P (D₂O) 18.43 (d, J=11.31Hz), 7.8 (dd), -10.96 (d, J=26.3Hz).
iii) 2-Propylthioadenosine. 5'-P¹ monoester with methylenebisphosphonic acid, P²-monoanhydride with phosphoric acid, tetrasodium salt
   The product of step ii) (0.3 g) in 0.4M triethylammonium bicarbonate solution (100 ml) containing semicarbazide hydrochloride (0.3 g) at 10° was irradiated with a high pressure UV lamp for 2 hours. The solvent was removed *in vacuo* at 30° and the residue purified by chromatography on DEAE-Sepharose eluted with 0-0.6M triethylammonium bicarbonate buffer. The relevant fractions were combined and lyophilised and the residue converted to the tetrasodium salt by the method of Example 5iii) (1.13 g).
   NMR δ³¹P (D₂O) 19.34 (d, J=8.56Hz), 6.7 (dd), -6.6 (d, J=20.96Hz).

### Example 7

### 3-(6-Amino-2-propylthio-9H-purin-9-yl)-5-(hydroxymethyl)-1,2-cyclopentanediol 5-dihydrogen phosphate, monoanhydride with dichloromethylenebisphosphonic acid, trisodium salt

i) 2-Propylthio-pyrimidine-4,6-diol
   To a solution of 4,6-dihydroxy-2-mercaptopyrimidine (100 g) in potassium hydroxide (2.5N, 571 ml) was added propyl iodide (76.8 ml) and the whole was stirred for 4 days. The solution was acidified to pH 2-3 and filtered to afford the sub-title compound (15 g).
   MS 330 M⁺ (di-TMS), BP 315.
ii) 5-Nitro-2-propylthiopyrimidine-4,6-diol
   The product of step i) (2 g) was added to ice-cooled fuming nitric acid (10 ml) over 1 hour and stirred at 0° for a further 1 hour. Pouring into ice followed by drying afforded the sub-title compound as a beige solid (1.7 g).
   MS 375 M⁺ (di-TMS), BP 360.
iii) 4,6-Dichloro-5-nitro-2-propylthiopyrimidine
   The product of step ii) (1.7 g), phosphoryl chloride (10 ml) and N,N-diethylaniline (3 ml) were heated to reflux for 1 hour, then concentrated to half volume and poured onto ice to yield a black tar. Extraction of the tar with ether afforded a solution which was dried (MgSO₄) then evaporated. The residue was chromatographed (SiO₂, light petrol) to afford the sub-title compound (0.8 g).
   MS 267/269/271 M⁺, BP 41.
iv) 4,6-Dichloro-2-propylthio-pyrimidine-5-amine
   To a solution of the product of step iii) (1 g) in glacial acetic acid (10 ml) was added reduced iron powder (1.1 g). The temperature was reduced to 60° by cooling and then heated at 60° for 15 min. The reaction was evaporated to dryness and the residue extracted into ether (100 ml), washed with dilute sodium hydroxide solution and dried. Evaporation afforded the sub-title compound as a dark oil (0.8 g).
   NMR δ¹H (CDCl₃) 4.2 (brs, 2H), 3.1 (t, 2H), 1.71 (q, 2H), 1.05 (t, 3H).
v) 3-(5-Amino-6-chloro-2-propylthiopyrimidine-4-ylamino)-5-(hydroxymethyl)cyclopentane-1,2-diol
   The product of step iv) (4.6 g) was added to a solution of 3-amino-5-(hydroxymethyl)-cyclopentane-1,2-diol (1.41 g) in butan-1-ol (200 ml) containing triethylamine (10 ml) and the whole heated to reflux for 48 hours. Evaporation gave a dark residue which was chromatographed (SiO₂, 10% ethanol/dichloromethane) to afford the sub-title compound (2.8 g).
   MS 349/350 (M⁺+H), BP 349.
vi) 3-(6-Amino-2-propylthio-9H-purin-9-yl)-5-(hydroxymethyl)-1,2-cyclopentanediol
   The product of step v) (0.2 g) and diethoxymethyl acetate (10 ml) were stirred at ambient temperature under nitrogen for 1 hour, then at 80° for 24 hours.
   Evaporation afforded a residue which was treated with liquid ammonia (15 ml) in an autoclave at 60° for 16 hours. The ammonia was allowed to evaporate and the residue taken up in 0.5M hydrochloric acid (10 ml) at 60° for 45 min. The volatiles were removed *in vacuo* and the remaining material dissolved in water (10 ml). Upon neutralisation with concentrated ammonia the sub-title compound crystallised and was collected and dried (0.15 g).
   MS (FAB) 340 (M⁺+H), BP 340.
vii) 3-(6-Amino-2-(propylthio)-9H-purin-9-yl)-5-[(phosphonooxy)methyl]-1,2-cyclopentanediol
   The product of step vi) was converted to the sub-title compound by the method of Example 1ii).
   NMR δ³¹P (D₂O) 2.85 (s).
viii) 3-(6-Amino-2-propylthio-9H-purin-9-yl)-5-(hydroxymethyl)-1,2-cyclopentanediol 5-dihydrogen phosphate, monoanhydride with dichloromethylenebisphosphonic acid, trisodium salt
   The product of step vii) was converted to the title salt by the method of Example 1.
   NMR δ³¹P (D₂O) 8.54 (d, J=18.73Hz), 0.84 (dd), -9.5 (d, J=29.61Hz).

### Example 8

### 1-(6-Methoxy-2-propylthio-purin-9-yl)-ribofuranos-5'-yl-phosphate, monoanhydride with dichloromethylenebisphosphonic acid, trisodium salt

i) 6-Chloro-2-(propylthio)-9-(2,3,5-tri-O-acetyl-β-D-ribofuranosyl)-9H-purine
   The sub-title compound was prepared according to the method of Example 3i), using di-n-propyl disulphide.
   NMR δ¹H (CDCl₃) 8.13 (s, 1H), 6.14 (d, 1H), 5.93 (t, 1H), 5.60 (t, 1H), 4.3-4.5 (m, 3H), 3.21 (t, 2H), 2.15, 2.13, 2.11 (3xs, 3x3H), 1.8 (m, 2H), 1.07 (t, 3H).
ii) 6-Methoxy-2-propylthio-9-ribofuranosyl-purine
   The compound from step i) (5.2 g) was dissolved in 1M sodium methoxide in methanol solution (53 ml) and heated to reflux for 1 hour under nitrogen. Dilution with water (100 ml), neutralisation with dilute hydrochloric acid and evaporation of the methanol gave an oil. Trituration with water and then dichloromethane afforded the sub-title compound as a colourless solid (1.35 g).
   MS (FAB) 357 (M⁺+H), BP 225.
iii) 1-(6-Methoxy-2-propylthio-purin-9-yl)-ribofuranos-5'-yl-phosphate
   The sub-title compound was prepared according to the method of Example 1.
   NMR δ³¹P (D₂O) 1.69 (s).
iv) 1-(6-Methoxy-2-propylthio-purin-9-yl)-ribofuranos-5'-yl-phosphate, monoanhydride with dichloromethylenebisphosphonic acid, trisodium salt
   The title salt was prepared according to the method of Example 1.
   NMR δ³¹P (D₂O) 8.59 (d, J=18Hz), 1.73 (dd, J=18 and 29Hz), -9.94 (d, J=29Hz).

### Example X

### Quantification of P_{2T} receptor agonist/antagonist activity in washed human platelets.

### Preparation

Human venous blood (100 ml) was divided equally between 3 tubes, each containing 3.2% trisodium citrate (4 ml) as anti-coagulant. The tubes were centrifuged for 15 min at 240G to obtain a platelet-rich plasma (PRP) to which 300 ng/ml prostacyclin (PGI₂, 3µ230l/ml PRP of 1/10 diln. in saline from stock 1 mg/ml in ethanol) was added to stabilize the platelets during the washing procedure. Red cell free PRP was obtained by centrifugation for 10 min at 125G followed by further centrifugation for 15 min at 640G. The supernatant was discarded and the platelet pellet resuspended in modified, calcium free, Tyrode solution ((10 ml) CFT, composition: NaCl 137mM (8 g/l), NaHCO₃ 11.9mM (1 g/l), NaH₂PO₄ 0.38mM (0.06 g/l), KCl 2.86mM (1 ml of 20% soln./l), MgCl₂ 1.05 mM (1 ml of 10% soln./l), dextrose 5.55mM (1 g/l)), gassed with 95% O₂/5% CO₂ and maintained at 37°. Following addition of a further 300 ng/ml PGI₂, the pooled suspension was centrifuged once more for 15 min at 640G. The supernatant was discarded and the platelets resuspended initially in 10 ml CFT with further CFT added to adjust the final platelet count to 2x10⁵/µl. This final suspension was stored in a 60 ml syringe at 3° with air excluded.

To allow recovery from PGI₂-inhibition of normal function, platelets were used in aggregation studies no sooner than 2 hours after final resuspension. In all studies 430 µl aliquots of platelet suspension were added to siliconized aggregation cuvettes containing CaCl₂ solution (10 µl of 45 mM soln., final conc. 1mM) and stirred at 900 rpm in a PAP4 aggregometer (Biodata). Human fibrinogen (Sigma, F 4883) and 8-sulphophenyl-theophylline (8-SPT, to block any P₁ agonist activity of compounds) were added to give final concentrations of 0.2 mg/ml (10 µl of 10 mg/ml solution of clottable protein in saline) and 3x10⁻⁴M (10 µl of 5.6 mg/ml solution in 6% glucose), respectively. Recording of aggregation was then started.

### Protocol

a) Selection of submaximal ADP concentration
   A concentration of ADP producing a just submaximal response was selected by constructing a concentration/response curve over the range 10-300µM. The appropriate solution of ADP was added to the aggregation cuvette in a volume of 10 µl, 20 min after starting the aggregation trace. Aggregation responses were measured using the maximum rate of change in light transmission, an index given by the PAP4 slope-reader. The submaximal concentration of ADP selected at this stage of the protocol was used in the subsequent assessment of antagonist potency of the compounds. All measurements were made in duplicate in platelets from each donor.
b) Assessment of agonist/antagonist potency
   5 min after starting the aggregation trace, saline or the appropriate solution of test compound was added to an aggregation cuvette in a volume of 30 µl to give a final concentration of 0, 10, 100 or 1000 µM. Aggregation at this point was indicative of agonist activity and, if it occurred, agonist potency was estimated by comparison with control ADP responses obtained in a). If aggregation did not occur the previously selected submaximal concentration of ADP was added in a volume of 10 µl, 15 min after the test compound. Antagonist potency was estimated as a % inhibition of the control ADP response to obtain an approximate IC₅₀. Compounds which completely inhibited the ADP response at the initial concentrations were retested at a lower concentration range. Compounds with an IC₅₀ <10⁻⁸M were also retested in the absence of 8-SPT to confirm the lack of any P₁ agonist activity and with a 2 min rather than a 15 min incubation to check whether inhibition was time dependent.

### Results

Results are reported as the negative logarithm of the antagonist potency (pIC₅₀) obtained in duplicate form from each of 4 donors. For compounds with pIC₅₀ >8 a comment "clean" is made if there is no evidence of P₁ agonist activity. An IC₅₀ <3 is defined as "inactive".

## Claims (Claims for the following Contracting State(s): AT, BE, CH, DE, DK, FR, GB, IT, LI, LU, MC, NL, PT, SE)

1. Compounds of formula I, 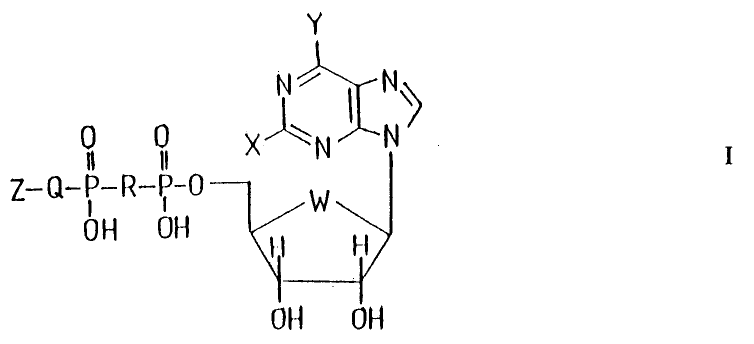 wherein Q represents CR¹R²,
R represents O or CR³R⁴,
W represents O or CH₂,
R¹, R², R³ and R⁴ independently represent hydrogen or halogen,
X represents S(O)ₙR⁵,
n represents 0, 1 or 2,
R⁵ represents ethyl, propyl or butyl,
Y represents NH₂ or alkoxy C₁₋₆, and
Z represents an acidic moiety,
in addition:
a) when R represents CR³R⁴, then -Q-Z may represent hydroxy or -OP(O)(OH)₂; and
b) when R and W represent O, Q represents CCl₂ or CBr₂, Y represents NH₂ and Z represents -P(O)(OH)₂, then X may represent S-pentyl;
and pharmaceutically acceptable salts thereof.

2. A compound of formula I, as defined in Claim 1, or a pharmaceutically acceptable salt thereof, wherein Z is -P(O)(OH)₂.

3. A compound of formula I, as defined in Claim 1 or 2, or a pharmaceutically acceptable salt thereof, wherein Q is CR¹R² and R is O.

4. A compound of formula I, as defined in any one of the preceding claims, or a pharmaceutically acceptable salt thereof, wherein W is O.

5. A compound of formula I, as defined in any one of the preceding claims, or a pharmaceutically acceptable salt thereof, wherein n is 0.

6. A compound of formula I, as defined in Claim 1, which is
2-Propylthio-5'-adenylic acid, monoanhydride with dichloromethylenebisphosphonic acid,
or a pharmaceutically acceptable salt thereof.

7. A compound of formula I, as defined in Claim 1, which is
2-Propylthio-5'-adenylic acid, monoanhydride with dibromomethylenebisphosphonic acid,
2-Propylthio-5'-adenylic acid, monoanhydride with difluoromethylenebisphosphonic acid,
2-Pentylthio-5'-adenylic acid, monoanhydride with dichloromethylenebisphosphonic acid,
2-Pentylthio-5'-adenylic acid, monoanhydride with dibromomethylenebisphosphonic acid,
2-Ethylthio-5'-adenylic acid, monoanhydride with dichloromethylenebisphosphonic acid,
2-Ethylthio-5'-adenylic acid, monoanhydride with dibromomethylenebisphosphonic acid,
2-Butylthio-5'-adenylic acid, monoanhydride with dichloromethylenebisphosphonic acid,
2-Propylthio-5'-adenylic acid, monoanhydride with methylenebisphosphonic acid,
2-Propylthio-5'-adenylic acid, monoanhydride with sulphodifluoromethylphosphonic acid,
2-Propylthio-5'-adenylic acid, monoanhydride with phosphonoacetic acid,
2-(1-Methylethyl)thio-5'-adenylic acid, monoanhydride with dichloromethylenebisphosphonic acid,
2-Propylthioadenosine, 5'-P¹ monoester with bis[(dihydroxyphosphinyl)methyl]phosphinic acid,
2-Propylthioadenosine, 5'-P¹ monoester with bis[(dichloro(dihydroxyphosphinyl))methyl]phosphinic acid,
2-Propylthioadenosine, 5'-P¹ monoester with methylenebisphosphonic acid, P²-monoanhydride with phosphoric acid,
2-Propylthioadenosine, 5'-P¹ monoester with methylenebisphosphonic acid,
3-(6-Amino-2-propylthio-9H-purin-9-yl)-5-(hydroxymethyl)-1,2-cyclopentanediol 5-dihydrogen phosphate, monoanhydride with dichloromethylenebisphosphonic acid,
1-(6-Methoxy-2-propylthio-purin-9-yl)-ribofuranos-5'-yl-phosphate, monoanhydride with dichloromethylenebisphosphonic acid,
or a pharmaceutically acceptable salt of any one thereof.

8. A pharmaceutical composition comprising a compound of formula I, as defined in any one of the preceding Claims, or a pharmaceutically acceptable salt thereof, in admixture with a pharmaceutically acceptable carrier, diluent or adjuvant.

9. A process for the preparation of a compound of formula I, as defined in any one of Claims 1-7, or a pharmaceutically acceptable salt thereof, which comprises
a) producing a compound of formula I in which R represents O, or a salt thereof, by reacting a compound of formula II, or a salt thereof 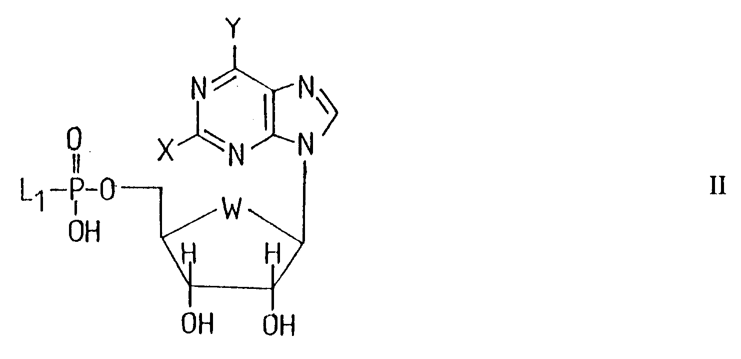 wherein W, X and Y are as defined in Claim 1 and L₁ represents a leaving group, with a compound of formula III, or a salt thereof, 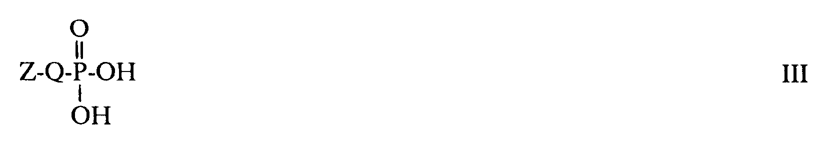 wherein Z and Q are as defined in Claim 1.
b) producing a compound of formula I in which R represents CR³R⁴, or a salt thereof, by reacting a compound of formula IV, or a salt thereof, 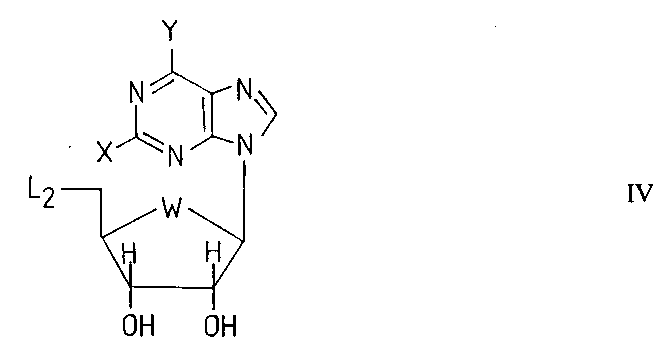 wherein W, X and Y are as defined in Claim 1 and L₂ represents a leaving group, with a compound of formula V, or a salt thereof, 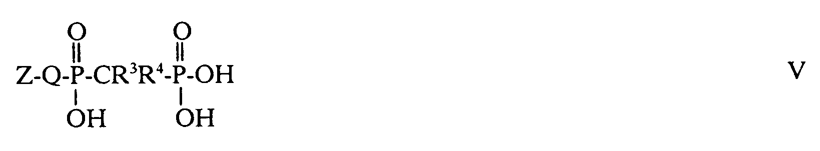 wherein Z, Q, R³ and R⁴ are as defined in Claim 1.
c) producing a compound of formula I in which R is CR³R⁴ and -Q-Z is -OP(O)(OH)₂, or a salt thereof, by reacting a corresponding compound of formula I in which -Q-Z is hydroxy with a compound of formula VI, or a salt thereof, 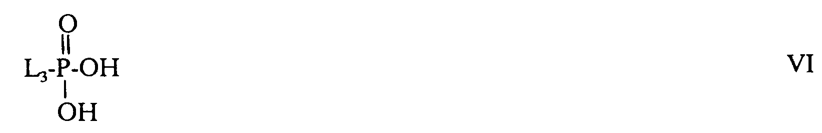 wherein L₃ is a leaving group.
d) removal of a protecting group from a corresponding protected compound of formula I in which one or more of the functional groups is protected,
and where desired or necessary converting the resulting compound of formula I, or another salt thereof, to a pharmaceutically acceptable salt thereof or vice versa.

## Claims (Claims for the following Contracting State(s): ES, GR)

1. A process for the production of a compound of formula I, 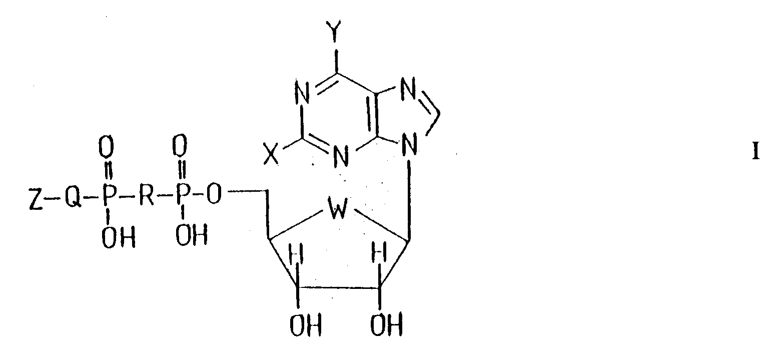 wherein Q represents CR¹R²,
R represents O or CR³R⁴,
W represents O or CH₂,
R¹, R², R³ and R⁴ independently represent hydrogen or halogen,
X represents S(O)ₙR⁵,
n represents 0, 1 or 2,
R⁵ represents ethyl, propyl or butyl,
Y represents NH₂ or alkoxy C₁₋₆, and
Z represents an acidic moiety,
in addition:
a) when R represents CR³R⁴, then -Q-Z may represent hydroxy or -OP(O)(OH)₂; and
b) when R and W represent O, Q represents CCl₂ or CBr₂, Y represents NH₂ and Z represents -P(O)(OH)₂, then X may represent S-pentyl;
or pharmaceutically acceptable salt thereof, which process comprises:
a) producing a compound of formula I in which R represents O, or a salt thereof, by reacting a compound of formula II, or a salt thereof 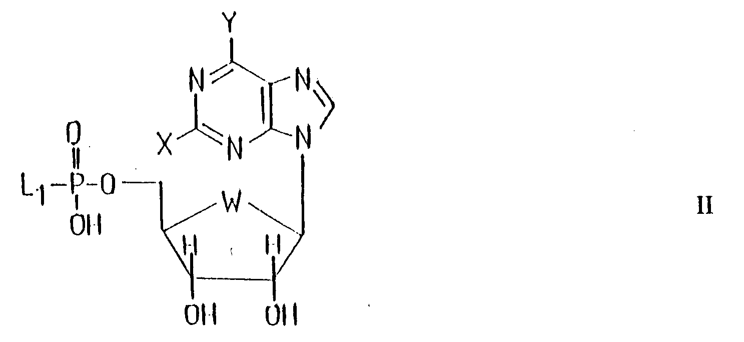 wherein W, X and Y are as defined as above and L₁ represents a leaving group, with a compound of formula III, or a salt thereof, 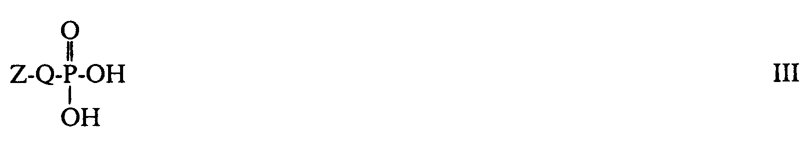 wherein Z and Q are as defined as above
b) producing a compound of formula I in which R represents CR³R⁴, or a salt thereof, by reacting a compound of formula IV, or a salt thereof, 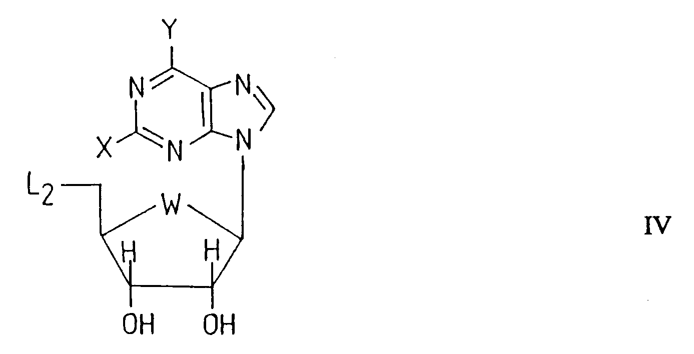 wherein W, X and Y are as defined as above and L₂ represents a leaving group, with a compound of formula V, or a salt thereof, 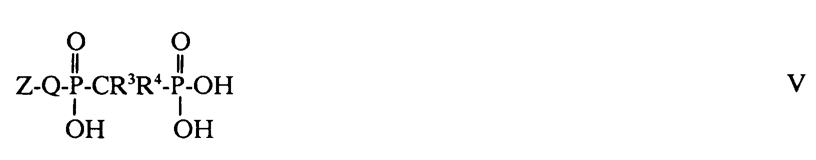 wherein Z, Q, R³ and R⁴ are as defined as above
c) producing a compound of formula I in which R is CR³R⁴ and -Q-Z is -OP(O)(OH)₂, or a salt thereof, by reacting a corresponding compound of formula I in which -Q-Z is hydroxy with a compound of formula VI, or a salt thereof, 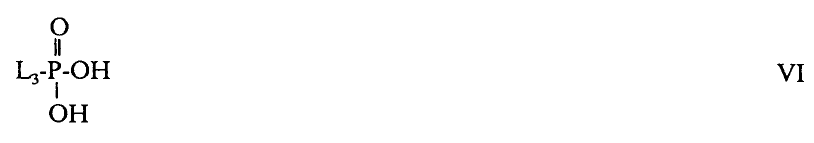 wherein L₃ is a leaving group.
d) removal of a protecting group from a corresponding protected compound of formula I in which one or more of the functional groups is protected,
and where desired or necessary converting the resulting compound of formula I, or another salt thereof, to a pharmaceutically acceptable salt thereof or vice versa.

2. A process as defined in Claim 1, wherein Z is -P(O)(OH)₂.

3. A process as defined in Claim 1 or 2, wherein Q is CR¹R² and R is O.

4. A process as defined in any one of the preceding claims, wherein W is O.

5. A process as defined in any one of the preceding claims, wherein n is 0.

6. A process as defined in Claim 1, wherein the compound of formula I is:
2-Propylthio-5'-adenylic acid, monoanhydride with dichloromethylenebisphosphonic acid,
or a pharmaceutically acceptable salt thereof.

7. A process as defined in Claim 1, wherein the compound of formula I is:
2-Propylthio-5'-adenylic acid, monoanhydride with dibromomethylenebisphosphonic acid,
2-Propylthio-5'-adenylic acid, monoanhydride with difluoromethylenebisphosphonic acid,
2-Pentylthio-5'-adenylic acid, monoanhydride with dichloromethylenebisphosphonic acid,
2-Pentylthio-5'-adenylic acid, monoanhydride with dibromomethylenebisphosphonic acid,
2-Ethylthio-5'-adenylic acid, monoanhydride with dichloromethylenebisphosphonic acid,
2-Ethylthio-5'-adenylic acid, monoanhydride with dibromomethylenebisphosphonic acid,
2-Butylthio-5'-adenylic acid, monoanhydride with dichloromethylenebisphosphonic acid,
2-Propylthio-5'-adenylic acid, monoanhydride with methylenebisphosphonic acid,
2-Propylthio-5'-adenylic acid, monoanhydride with sulphodifluoromethylphosphonic acid,
2-Propylthio-5'-adenylic acid, monoanhydride with phosphonoacetic acid,
2-(1-Methylethyl)thio-5'-adenylic acid, monoanhydride with dichloromethylenebisphosphonic acid,
2-Propylthioadenosine, 5'-P¹ monoester with bis[(dihydroxyphosphinyl)methyl]phosphinic acid,
2-Propylthioadenosine, 5'-P¹ monoester with bis[(dichloro(dihydroxyphosphinyl))methyl]phosphinic acid,
2-Propylthioadenosine, 5'-P¹ monoester with methylenebisphosphonic acid, P²-monoanhydride with phosphoric acid,
2-Propylthiocidenosine, 5'-P¹ monoester with methylenebisphosphonic acid,
3-(6-Amino-2-propylthio-9H-purin-9-yl)-5-(hydroxymethyl)-1,2-cyclopentanediol 5-dihydrogen phosphate, monoanhydride with dichloromethylenebisphosphonic acid,
1-(6-Methoxy-2-propylthio-purin-9-yl)-ribofuranos-5'-yl-phosphate, monoanhydride with dichloromethylenebisphosphonic acid,
or a pharmaceutically acceptable salt of any one thereof.

8. A process for the production of a pharmaceutical composition comprising a compound of formula I as defined in any one of the preceding claims, or a pharmaceutically acceptable salt thereof, which comprises mixing the compound of formula I with a pharmaceutically acceptable carrier, diluent or adjuvant.

## Patentansprüche (Patentansprüche für folgende(n) Vertragsstaat(en): AT, BE, CH, DE, DK, FR, GB, IT, LI, LU, MC, NL, PT, SE)

1. Verbindungen der Formel (I) 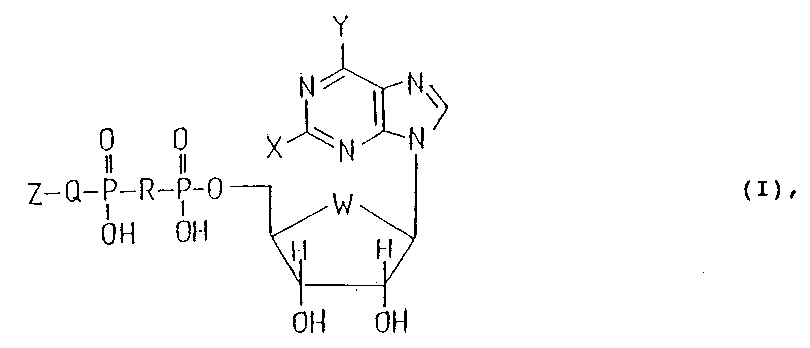 worin Q die Bedeutung CR¹R² hat, R O oder CR³R⁴ darstellt, W O oder CH₂ ist, R¹, R², R³ und R⁴ unabhängig Wasserstoff oder Halogen bedeuten, X S(O)ₙR⁵ darstellt, n Null, 1 oder 2 ist, R⁵ Ethyl, Propyl oder Butyl bedeutet, Y NH₂ oder C₁-C₆-Alkoxy darstellt, und Z eine Säuregruppe ist;
und außerdem
a) wenn R CR³R⁴ bedeutet, -Q-Z Hydroxy oder -OP(O)(OH)₂ darstellen kann; und
b) wenn R und W die Bedeutung O haben, Q CCl₂ oder CBr₂ darstellt, Y NH₂ ist, und Z -P(O)(OH)₂ bedeutet, X S-Pentyl sein kann;
und pharmazeutisch annehmbare Salze hievon.

2. Verbindung der Formel (I), wie in Anspruch 1 definiert, oder ein pharmazeutisch annehmbares Salz hievon, worin Z die Bedeutung -P(O)(OH)₂ hat.

3. Verbindung der Formel (I), wie in Anspruch 1 oder 2 definiert, oder ein pharmazeutisch annehmbares Salz hievon, worin Q CR¹R² ist, und R die Bedeutung O hat.

4. Verbindung der Formel (I), wie in einem der vorhergehenden Ansprüche definiert, oder ein pharmazeutisch annehmbares Salz hievon, worin W die Bedeutung O hat.

5. Verbindung der Formel (I), wie in einem der vorhergehenden Ansprüche definiert, oder ein pharmazeutisch annehmbares Salz hievon, worin n Null ist.

6. Verbindung der Formel (I), wie in Anspruch 1 definiert, nämlich
2-Propylthio-5'-adenylsäure, Monoanhydrid mit Dichlormethylenbisphosphonsäure,
oder ein pharmazeutisch annehmbares Salz hievon.

7. Verbindung der Formel (I), wie in Anspruch 1 definiert, nämlich
2-Propylthio-5'-adenylsäure, Monoanhydrid mit Dibrommethylenbisphosphonsäure,
2-Propylthio-5'-adenylsäure, Monoanhydrid mit Difluormethylenbisphosphonsäure,
2-Pentylthio-5'-adenylsäure, Monoanhydrid mit Dichlormethylenbisphosphonsäure,
2-Pentylthio-5'-adenylsäure, Monoanhydrid mit Dibrommethylenbisphosphonsäure,
2-Ethylthio-5'-adenylsäure, Monoanhydrid mit Dichlormethylenbisphosphonsäure,
2-Ethylthio-5'-adenylsäure, Monoanhydrid mit Dibrommethylenbisphosphonsäure,
2-Butylthio-5'-adenylsäure, Monoanhydrid mit Dichlormethylenbisphosphonsäure,
2-Propylthio-5'-adenylsäure, Monoanhydrid mit Methylenbisphosphonsäure,
2-Propylthio-5'-adenylsäure, Monoanhydrid mit Sulfodifluormethylphosphonsäure,
2-Propylthio-5'-adenylsäure, Monoanhydrid mit Phosphonoessigsäure,
2-(1-Methylethyl)-thio-5'-adenylsäure, Monoanhydrid mit Dichlormethylenbisphosphonsäure,
2-Propylthioadenosin, 5'-P¹-Monoester mit Bis-[(dihydroxyphosphinyl)-methyl]-phosphinsäure,
2-Propylthioadenosin, 5'-P¹-Monoester mit Bis-[(dichlor(dihydroxyphosphinyl)-methyl]-phosphinsäure,
2-Propylthioadenosin, 5'-P¹-Monoester mit Methylenbisphosphonsäure, P²-Monoanhydrid mit Phosphorsäure,
2-Propylthioadenosin, 5'-P¹-Monoester mit Methylenbisphosphonsäure,
3-(6-Amino-2-propylthio-9H-purin-9-yl)-5-(hydroxymethyl)-1,2-cyclopentandiol-5-dihydrogenphosphat, Monoanhydrid mit Dichlormethylenbisphosphonsäure,
1-(6-Methoxy-2-propylthiopurin-9-yl)-ribofuranos-5'-yl-phosphat, Monoanhydrid mit Dichlormethylenbisphosphonsäure,
oder ein pharmazeutisch annehmbares Salz hievon.

8. Pharmazeutische Zusammensetzung, welche eine Verbindung der Formel (I), wie in einem der vorhergehenden Ansprüche definiert, oder ein pharmazeutisch annehmbares Salz hievon in Mischung mit einem pharmazeutisch annehmbaren Träger, Verdünnungsmittel oder Adjuvans umfaßt.

9. Verfahren zur Herstellung einer Verbindung der Formel (I), wie in einem der Ansprüche 1 bis 7 definiert, oder eines pharmazeutisch annehmbaren Salzes hievon, welches umfaßt:
a) Herstellen einer Verbindung der Formel (I), worin R die Bedeutung O hat, oder eines Salzes hievon durch das Umsetzen einer Verbindung der Formel (II) oder eines Salzes hievon 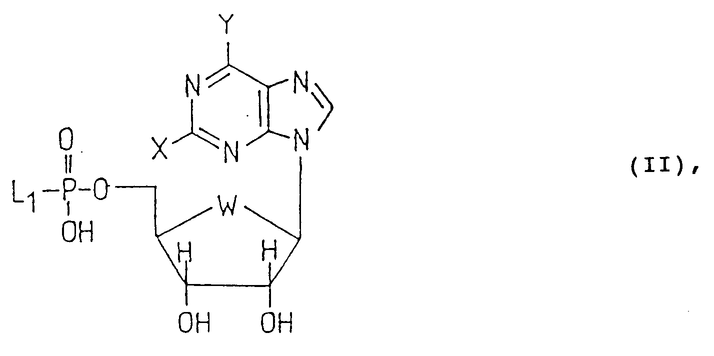 worin W, X und Y wie in Anspruch 1 definiert sind, und L₁ eine Abgangsgruppe darstellt, mit einer Verbindung der Formel (III) oder einem Salz hievon 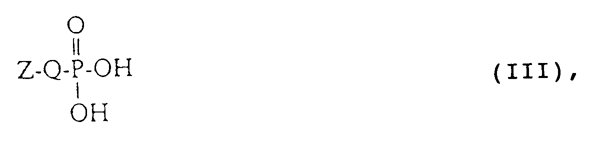 worin Z und Q wie in Anspruch 1 definiert sind;
b) Herstellen einer Verbindung der Formel (I), worin R die Bedeutung CR³R⁴ hat, oder eines Salzes hievon durch das Umsetzen einer Verbindung der Formel (IV) oder eines Salzes hievon 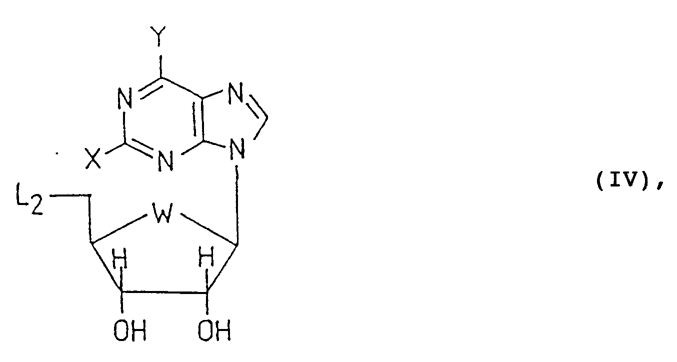 worin W, X und Y wie in Anspruch 1 definiert sind, und L₂ eine Abgangsgruppe darstellt, mit einer Verbindung der Formel (V) oder einem Salz hievon 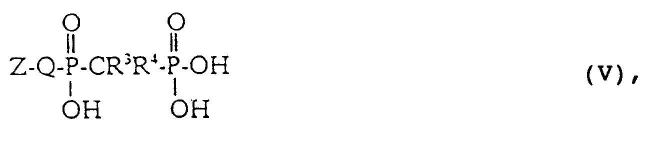 worin Z, Q, R³ und R⁴ wie in Anspruch 1 definiert sind;
c) Herstellen einer Verbindung der Formel (I), worin R die Bedeutung CR³R⁴ hat, und -Q-Z -OP(O)(OH)₂ darstellt, oder eines Salzes hievon durch das Umsetzen einer entsprechenden Verbindung der Formel (I), worin -Q-Z Hydroxy bedeutet, mit einer Verbindung der Formel (VI) oder einem Salz hievon 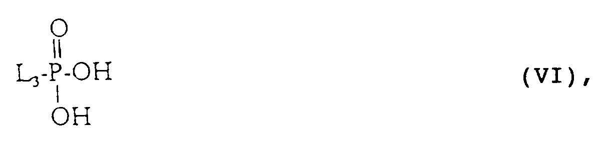 worin L₃ eine Abgangsgruppe ist;
d) Entfernen einer Schutzgruppe aus einer entsprechenden geschützten Verbindung der Formel (I), worin eine oder mehrere der funktionellen Gruppen geschützt sind,
und, wenn gewünscht oder notwendig, Überführen der erhaltenen Verbindung der Formel (I) oder eines anderen Salzes hievon in ein pharmazeutisch annehmbares Salz hievon oder umgekehrt.

## Patentansprüche (Patentansprüche für folgende(n) Vertragsstaat(en): ES, GR)

1. Verfahren zur Herstellung einer Verbindung der Formel (I) 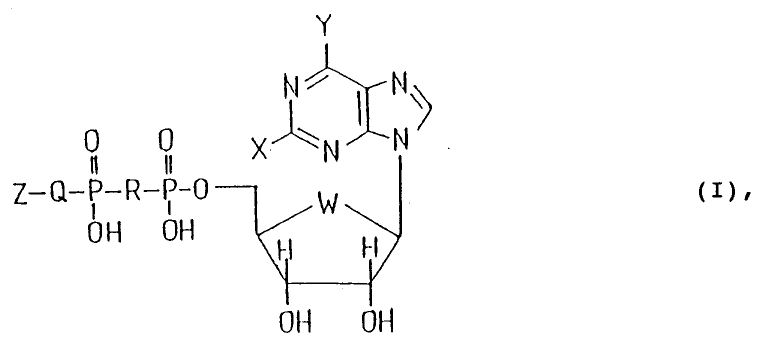 worin Q die Bedeutung CR¹R² hat, R O oder CR³R⁴ darstellt, W O oder CH₂ ist, R¹, R², R³ und R⁴ unabhängig Wasserstoff oder Halogen bedeuten, X S(O)ₙR⁵ darstellt, n Null, 1 oder 2 ist, R⁵ Ethyl, Propyl oder Butyl bedeutet, Y NH₂ oder C₁-C₆-Alkoxy darstellt, und Z eine Säuregruppe ist; und außerdem
a) wenn R CR³R⁴ bedeutet, -Q-Z Hydroxy oder -OP(O)(OH)₂ darstellen kann; und
b) wenn R und W die Bedeutung O haben, Q CCl₂ oder CBr₂ darstellt, Y NH₂ ist, und Z -P(O)(OH)₂ bedeutet, X S-Pentyl sein kann;
oder eines pharmazeutisch annehmbaren Salzes hievon, welches Verfahren umfaßt:
a) Herstellen einer Verbindung der Formel (I), worin R die Bedeutung O hat, oder eines Salzes hievon durch das Umsetzen einer Verbindung der Formel (II) oder eines Salzes hievon 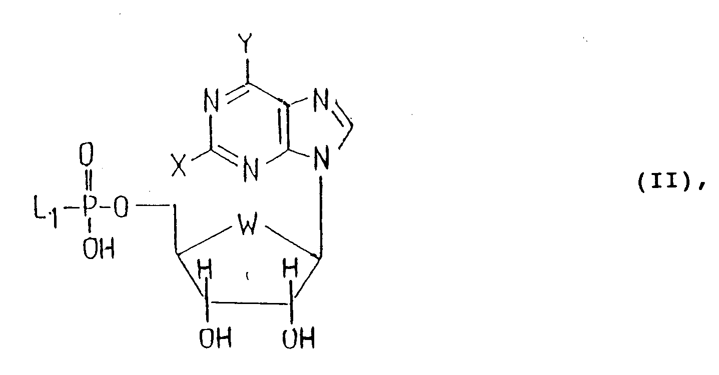 worin W, X und Y wie oben definiert sind, und L₁ eine Abgangsgruppe darstellt, mit einer Verbindung der Formel (III) oder einem Salz hievon 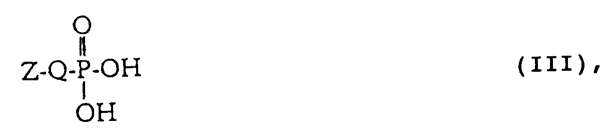 worin Z und Q wie oben definiert sind;
b) Herstellen einer Verbindung der Formel (I), worin R die Bedeutung CR³R⁴ hat, oder eines Salzes hievon durch das Umsetzen einer Verbindung der Formel (IV) oder eines Salzes hievon 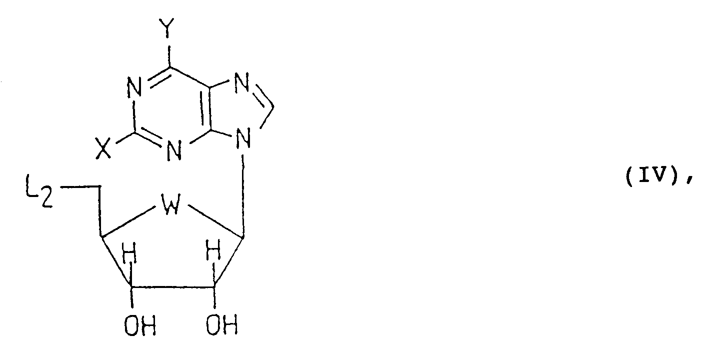 worin W, X und Y wie oben definiert sind, und L₂ eine Abgangsgruppe darstellt, mit einer Verbindung der Formel (V) oder einem Salz hievon 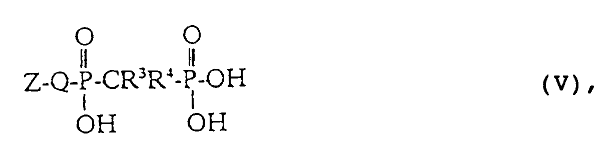 worin Z, Q, R³ und R⁴ wie oben definiert sind;
c) Herstellen einer Verbindung der Formel (I), worin R die Bedeutung CR³R⁴ hat, und -Q-Z -OP(O)(OH)₂ darstellt, oder eines Salzes hievon durch das Umsetzen einer entsprechenden Verbindung der Formel (I), worin -Q-Z Hydroxy bedeutet, mit einer Verbindung der Formel (VI) oder einem Salz hievon 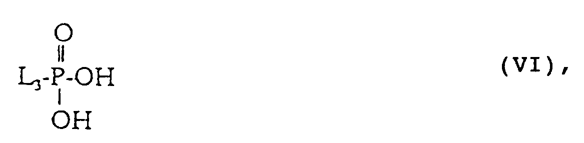 worin L₃ eine Abgangsgruppe ist;
d) Entfernen einer Schutzgruppe aus einer entsprechenden geschützten Verbindung der Formel (I), worin eine oder mehrere der funktionellen Gruppen geschützt sind,
und, wenn gewünscht oder notwendig, Überführen der erhaltenen Verbindung der Formel (I) oder eines anderen Salzes hievon in ein pharmazeutisch annehmbares Salz hievon oder umgekehrt.

2. Verfahren, wie in Anspruch 1 definiert, wobei Z die Bedeutung -P(O)(OH)₂ hat.

3. Verfahren, wie in Anspruch 1 oder 2 definiert, wobei Q CR¹R² ist, und R die Bedeutung O hat.

4. Verfahren, wie in einem der vorhergehenden Ansprüche definiert, wobei W die Bedeutung O hat.

5. Verfahren, wie in einem der vorhergehenden Ansprüche definiert, oder wobei n Null ist.

6. Verfahren, wie in Anspruch 1 definiert, bei welchem die Verbindung der Formel (I) ist:
2-Propylthio-5'-adenylsäure, Monoanhydrid mit Dichlormethylenbisphosphonsäure,
oder ein pharmazeutisch annehmbares Salz hievon.

7. Verfahren, wie in Anspruch 1 definiert, bei welchem die Verbindung der Formel (I) ist:
2-Propylthio-5'-adenylsäure, Monoanhydrid mit Dibrommethylenbisphosphonsäure,
2-Propylthio-5'-adenylsäure, Monoanhydrid mit Difluormethylenbisphosphonsäure,
2-Pentylthio-5'-adenylsäure, Monoanhydrid mit Dichlormethylenbisphosphonsäure,
2-Pentylthio-5'-adenylsäure, Monoanhydrid mit Dibrommethylenbisphosphonsäure,
2-Ethylthio-5'-adenylsäure, Monoanhydrid mit Dichlormethylenbisphosphonsäure,
2-Ethylthio-5'-adenylsäure, Monoanhydrid mit Dibrommethylenbisphosphonsäure,
2-Butylthio-5'-adenylsäure, Monoanhydrid mit Dichlormethylenbisphosphonsäure,
2-Propylthio-5'-adenylsäure, Monoanhydrid mit Methylenbisphosphonsäure,
2-Propylthio-5'-adenylsäure, Monoanhydrid mit Sulfodifluormethylphosphonsäure,
2-Propylthio-5'-adenylsäure, Monoanhydrid mit Phosphonoessigsäure,
2-(1-Methylethyl)-thio-5'-adenylsäure, Monoanhydrid mit Dichlormethylenbisphosphonsäure,
2-Propylthioadenosin, 5'-P¹-Monoester mit Bis-[(dihydroxyphosphinyl)-methyl]-phosphinsäure,
2-Propylthioadenosin, 5'-P¹-Monoester mit Bis-[(dichlor(dihydroxyphosphinyl)-methyl]-phosphinsäure,
2-Propylthioadenosin, 5'-P¹-Monoester mit Methylenbisphosphonsäure, P²-Monoanhydrid mit Phosphorsäure,
2-Propylthioadenosin, 5'-P¹-Monoester mit Methylenbisphosphonsäure,
3-(6-Amino-2-propylthio-9H-purin-9-yl)-5-(hydroxymethyl)-1,2-cyclopentandiol-5-dihydrogenphosphat, Monoanhydrid mit Dichlormethylenbisphosphonsäure,
1-(6-Methoxy-2-propylthiopurin-9-yl)-ribofuranos-5'-yl-phosphat, Monoanhydrid mit Dichlormethylenbisphosphonsäure,
oder ein pharmazeutisch annehmbares Salz einer hievon.

8. Verfahren zur Herstellung einer pharmazeutischen Zusammensetzung, die eine Verbindung der Formel (I), wie in einem der vorhergehenden Ansprüche definiert, oder ein pharmazeutisch annehmbares Salz hievon umfaßt, welches das Mischen der Verbindung der Formel (I) mit einem pharmazeutisch annehmbaren Träger, Verdünnungsmittel oder Adjuvans umfaßt.

## Revendications (Revendications pour l'(les) Etat(s) contractant(s) suivant(s): AT, BE, CH, DE, DK, FR, GB, IT, LI, LU, MC, NL, PT, SE)

1. Composés de formule (I) : 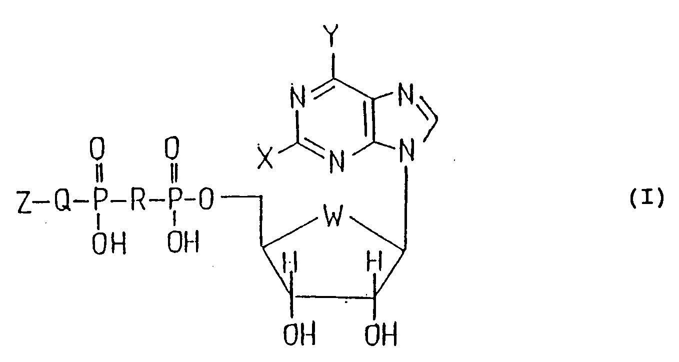 dans lesquels :
Q représente CR¹R²;
R représente O ou CR³R⁴;
W représente O ou CH₂;
R¹, R², R³ et R⁴ représentent, indépendamment, hydrogène ou halogène;
X représente S(O)ₙR⁵;
n représente 0, 1 ou 2;
R⁵ représente éthyle, propyle ou butyle;
Y représente NH₂ ou alcoxy en C₁₋₆, et
Z représente un résidu acide,
en outre :
a) lorsque R représente CR³R⁴, alors -Q-Z peut représenter hydroxyle ou -OP(O)(OH)₂, et
b) lorsque R et W représentent O, Q représente CCl₂ ou CBr₂, Y représente NH₂ et Z représente -P(O)(OH)₂, alors X peut représenter -S-pentyle;
et les sels pharmaceutiquement acceptables de ceux-ci.

2. Composé de formule (I), tel que défini à la revendication 1, ou un sel pharmaceutiquement acceptable de celui-ci, dans lequel Z est -P(O)(OH)₂.

3. Composé de formule (I), tel que défini à la revendication 1 ou 2, ou un sel pharmaceutiquement acceptable de celui-ci, dans lequel Q est CR¹R² et R est O.

4. Composé de formule (I), tel que défini dans l'une quelconque des revendications précédentes, ou un sel pharmaceutiquement acceptable de celui-ci, dans lequel W est O.

5. Composé de formule (I), tel que défini dans l'une quelconque des revendications précédentes, ou un sel pharmaceutiquement acceptable de celui-ci, dans lequel n est 0.

6. Composé de formule (I), tel que défini à la revendication 1, qui est le monoanhydride de l'acide 2-propylthio-5'-adénylique avec l'acide dichlorométhylènebisphosphonique, ou un sel pharmaceutiquement acceptable de celui-ci.

7. Composé de formule (I), tel que défini à la revendication 1, qui est :
le monoanhydride de l'acide 2-propylthio-5'-adénylique avec l'acide dibromométhylènebisphosphonique;
le monoanhydride de l'acide 2-propylthio-5'-adénylique avec l'acide difluorométhylènebisphosphonique;
le monoanhydride de l'acide 2-pentylthio-5'-adénylique avec l'acide dichlorométhylènebisphosphonique;
le monoanhydride de l'acide 2-pentylthio-5'-adénylique avec l'acide dibromométhylènebisphosphonique;
le monoanhydride de l'acide 2-éthylthio-5'-adénylique avec l'acide dichlorométhylènebisphosphonique;
le monoanhydride de l'acide 2-éthylthio-5'-adénylique avec l'acide dibromométhylènebisphosphonique;
le monoanhydride de l'acide 2-butylthio-5'-adénylique avec l'acide dichlorométhylènebisphosphonique;
le monoanhydride de l'acide 2-propylthio-5'-adénylique avec l'acide méthylènebisphosphonique;
le monoanhydride de l'acide 2-propylthio-5'-adénylique avec l'acide sulfodifluorométhylphosphonique;
le monoanhydride de l'acide 2-propylthio-5'-adénylique avec l'acide phosphonoacétique;
le monoanhydride de l'acide 2-(1-méthyléthyl)thio-5'-adénylique avec l'acide dichlorométhylènebisphosphonique;
le monoester 5'-P¹ de la 2-propylthioadénosine avec l'acide bis[(dihydroxyphosphinyl)méthyl]phosphinique;
le monoester 5'-P¹ de la 2-propylthioadénosine avec l'acide bis[(dichloro(dihydroxyphosphinyl))méthyl]phosphinique;
le monoester 5'-P¹ de la 2-propylthioadénosine avec l'acide méthylènebisphosphonique, monoanhydride en P² avec l'acide phosphorique;
le monoester 5'-P¹ de la 2-propylthioadénosine avec l'acide méthylènebisphosphonique;
le monoanhydride du dihydrogénophosphate de 3-(6-amino-2-propylthio-9H-purin-9-yl)-5-(hydroxyméthyl)-1,2-cyclopentanediol-5 avec l'acide dichlorométhylènebisphosphonique;
le monoanhydride du phosphate de 1-(6-méthoxy-2-propylthiopurin-9-yl)ribofurannos-5'-yle avec l'acide dichlorométhylènebisphosphonique, ou
un sel pharmaceutiquement acceptable de l'un quelconque de ceux-ci.

8. Composition pharmaceutique comprenant un composé de formule (I), tel que défini dans l'une quelconque des revendications précédentes, ou un sel pharmaceutiquement acceptable de celui-ci, en mélange avec un excipient, diluant ou adjuvant pharmaceutiquement acceptable.

9. Procédé de préparation d'un composé de formule (I), tel que défini dans l'une quelconque des revendications 1 à 7, ou d'un sel pharmaceutiquement acceptable de celui-ci, qui comprend :
a) la préparation d'un composé de formule (I), dans lequel R représente O, ou l'un de ses sels, par réaction d'un composé de formule (II), ou l'un de ses sels : 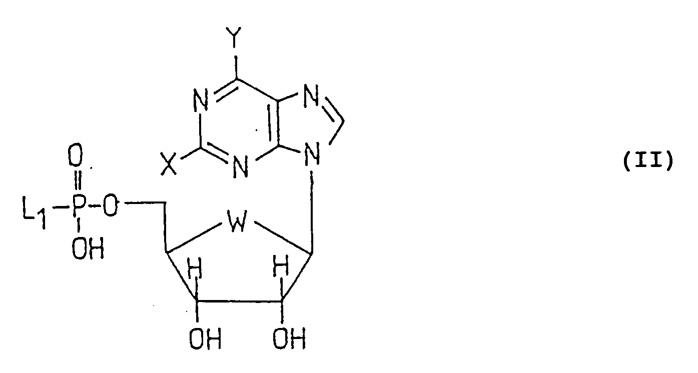 dans lequel :
W, X et Y sont tels que définis à la revendication 1, et
L₁ représente un groupe partant,
avec un composé de formule (III), ou l'un de ses sels : 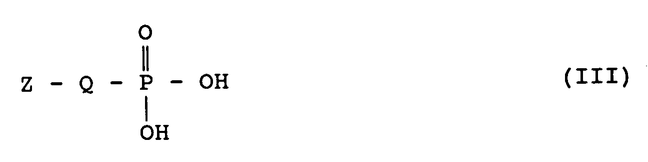 dans lequel :
Z et Q sont tels que définis à la revendication 1;
b) la préparation d'un composé de formule (I), dans lequel R représente CR³R⁴, ou l'un de ses sels, par réaction d'un composé de formule (IV), ou l'un de ses sels : 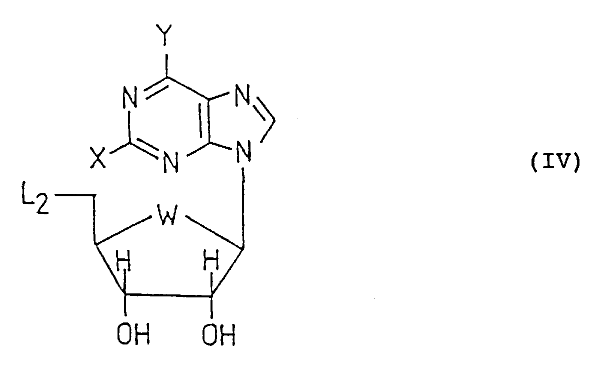 dans lequel :
W, X et Y sont tels que définis à la revendication 1, et
L₂ représente un groupe partant,
avec un composé de formule (V), ou l'un de ses sels : 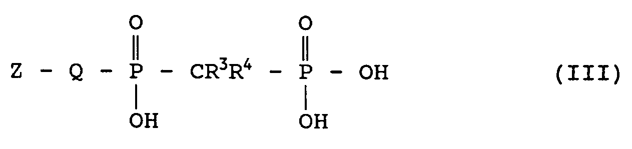 dans lequel :
Z, Q, R³ et R⁴ sont tels que définis à la revendication 1;
c) la préparation d'un composé de formule (I), dans lequel R représente CR³R⁴ et -Q-Z est -OP(O)(OH)₂, ou l'un de ses sels, par réaction d'un composé correspondant de formule (I), dans lequel -Q-Z est hydroxyle,
avec un composé de formule (VI), ou l'un de ses sels : 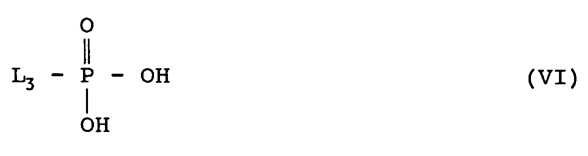 dans lequel :
L₃ est un groupe partant, ou
d) l'élimination d'un groupe protecteur d'un composé correspondant protégé de formule (I), dans lequel un ou plusieurs des groupes fonctionnels sont protégés, et si désiré ou nécessaire, conversion du composé résultant de formule (I), ou d'un autre de ses sels, en un sel pharmaceutiquement acceptable de celui-ci, ou vice versa.

## Revendications (Revendications pour l'(les) Etat(s) contractant(s) suivant(s): ES, GR)

1. Procédé de préparation d'un composé de formule (I) : 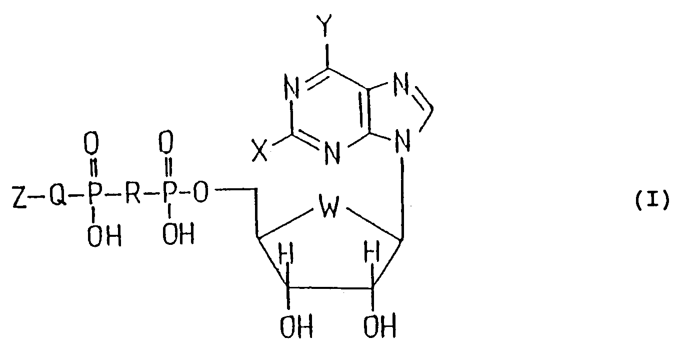 dans lequel :
Q représente CR¹R²;
R représente O ou CR³R⁴;
W représente O ou CH₂;
R¹, R², R³ et R⁴ représentent, indépendamment, hydrogène ou halogène;
X représente S(O)ₙR⁵;
n représente 0, 1 ou 2;
R⁵ représente éthyle, propyle ou butyle;
Y représente NH₂ ou alcoxy en C₁₋₆, et
Z représente un résidu acide,
en outre :
a) lorsque R représente CR³R⁴, alors -Q-Z peut représenter hydroxyle ou -OP(O)(OH)₂, et
b) lorsque R et W représentent O, Q représente CCl₂ ou CBr₂, Y représente NH₂ et Z représente -P(O)(OH)₂, alors X peut représenter -S-pentyle;
ou un sel pharmaceutiquement acceptable de celui-ci, le procédé comprenant :
a) la préparation d'un composé de formule (I), dans lequel R représente O, ou l'un de ses sels, par réaction d'un composé de formule (II), ou l'un de ses sels : 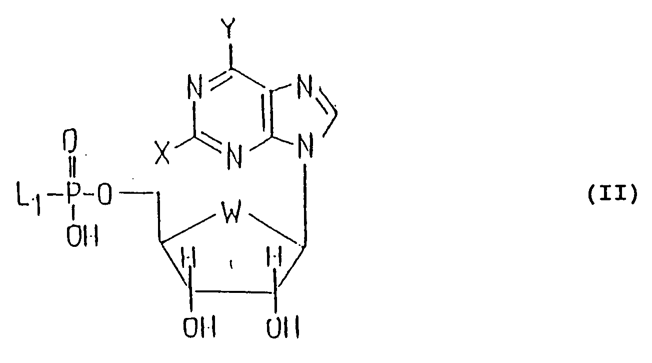 dans lequel :
W, X et Y sont tels que définis ci-dessus, et
L₁ représente un groupe partant,
avec un composé de formule (III), ou l'un de ses sels : 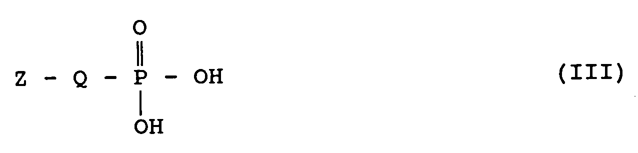 dans lequel :
Z et Q sont tels que définis ci-dessus;
b) la préparation d'un composé de formule (I), dans lequel R représente CR³R⁴, ou l'un de ses sels, par réaction d'un composé de formule (IV), ou l'un de ses sels : 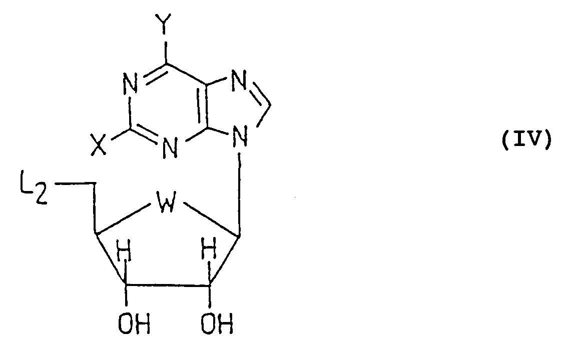 dans lequel :
W, X et Y sont tels que définis ci-dessus, et
L₂ représente un groupe partant,
avec un composé de formule (V), ou l'un de ses sels : 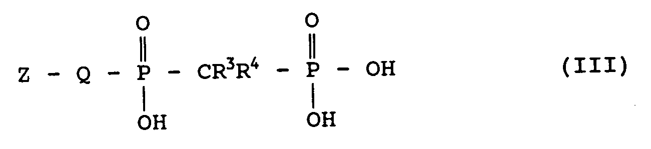 dans lequel :
Z, Q, R³ et R⁴ sont tels que définis ci-dessus;
c) la préparation d'un composé de formule (I), dans lequel R représente CR³R⁴ et -Q-Z est -OP(O)(OH)₂, ou l'un de ses sels, par réaction d'un composé correspondant de formule (I), dans lequel -Q-Z est hydroxyle,
avec un composé de formule (VI), ou l'un de ses sels : 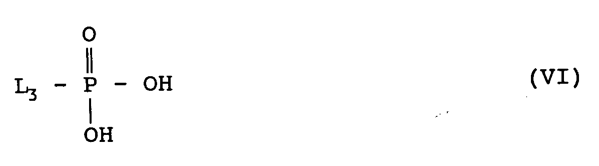 dans lequel :
L₃ est un groupe partant, ou
d) l'élimination d'un groupe protecteur d'un composé correspondant protégé de formule (I), dans lequel un ou plusieurs des groupes fonctionnels sont protégés, et si désiré ou nécessaire, conversion du composé résultant de formule (I), ou d'un autre de ses sels, en un sel pharmaceutiquement acceptable de celui-ci, ou vice versa.

2. Procédé tel que défini à la revendication 1, dans lequel Z est -P(O)(OH)₂.

3. Procédé tel que défini à la revendication 1 ou 2, dans lequel Q est CR¹R² et R est O.

4. Procédé tel que défini dans l'une quelconque des revendications précédentes, dans lequel W est O.

5. Procédé tel que défini dans l'une quelconque des revendications précédentes, dans lequel n est 0.

6. Procédé tel que défini à la revendication 1, dans lequel le composé de formule (I) est le monoanhydride de l'acide 2-propylthio-5'-adénylique avec l'acide dichlorométhylènebisphosphonique, ou un sel pharmaceutiquement acceptable de celui-ci.

7. Procédé tel que défini à la revendication 1, dans lequel le composé de formule (I) est :
le monoanhydride de l'acide 2-propylthio-5'-adénylique avec l'acide dibromométhylènebisphosphonique;
le monoanhydride de l'acide 2-propylthio-5'-adénylique avec l'acide difluorométhylènebisphosphonique;
le monoanhydride de l'acide 2-pentylthio-5'-adénylique avec l'acide dichlorométhylènebisphosphonique;
le monoanhydride de l'acide 2-pentylthio-5'-adénylique avec l'acide dibromométhylènebisphosphonique;
le monoanhydride de l'acide 2-éthylthio-5'-adénylique avec l'acide dichlorométhylènebisphosphonique;
le monoanhydride de l'acide 2-éthylthio-5'-adénylique avec l'acide dibromométhylènebisphosphonique;
le monoanhydride de l'acide 2-butylthio-5'-adénylique avec l'acide dichlorométhylènebisphosphonique;
le monoanhydride de l'acide 2-propylthio-5'-adénylique avec l'acide méthylènebisphosphonique;
le monoanhydride de l'acide 2-propylthio-5'-adénylique avec l'acide sulfodifluorométhylphosphonique;
le monoanhydride de l'acide 2-propylthio-5'-adénylique avec l'acide phosphonoacétique;
le monoanhydride de l'acide 2-(1-méthyléthyl)thio-5'-adénylique avec l'acide dichlorométhylènebisphosphonique;
le monoester 5'-P¹ de la 2-propylthioadénosine avec l'acide bis[(dihydroxyphosphinyl)méthyl]phosphinique;
le monoester 5'-P¹ de la 2-propylthioadénosine avec l'acide bis[(dichloro(dihydroxyphosphinyl))méthyl]phosphinique;
le monoester 5'-P¹ de la 2-propylthioadénosine avec l'acide méthylènebisphosphonique, monoanhydride en P² avec l'acide phosphorique;
le monoester 5'-P¹ de la 2-propylthioadénosine avec l'acide méthylènebisphosphonique;
le monoanhydride du dihydrogénophosphate de 3-(6-amino-2-propylthio-9H-purin-9-yl)-5-(hydroxyméthyl)-1,2-cyclopentanediol-5 avec l'acide dichlorométhylènebisphosphonique;
le monoanhydride du phosphate de 1-(6-méthoxy-2-propylthiopurin-9-yl)ribofurannos-5'-yle avec l'acide dichlorométhylènebisphosphonique, ou
un sel pharmaceutiquement acceptable de l'un quelconque de ceux-ci.

8. Procédé de préparation d'une composition pharmaceutique comprenant un composé de formule (I), tel que défini dans l'une quelconque des revendications précédentes, ou un sel pharmaceutiquement acceptable de celui-ci, qui comprend le mélange du composé de formule (I) avec un excipient, diluant ou adjuvant pharmaceutiquement acceptable.
